# EUROPEAN PATENT APPLICATION

(11) **EP 2 630 963 A1**
(43) Date of publication of application: **28.08.2013**
(21) Application number: 13166837.8
(22) Date of filing: 11.03.2011
(51) Int. Cl.: A61K 38/17, A61P 35/00

(54) **SPARC angiogenic domain and methods of use**

(30) Priority: 11.03.2010 US 313047 P; 11.03.2010 US 313050 P
(62) Divisional of application: 11754152.4
(71) Applicant: Abraxis BioScience, LLC, Los Angeles, CA 90025 (US)
(72) Inventor: Trieu, Vuong, Agoura Hills, CA 91301 (US); Knauer, Daniel, Costa Mesa, CA 92626 (US); Desai, Neil P., Los Angeles, CA 90025 (US)
(74) Representative: Grünecker, Kinkeldey, Stockmair & Schwanhäusser

(57) **Abstract**

The invention provides compositions and methods which exploit the discovery of the SPARC carboxy angiogenic domain.

## Description

### CROSS-REFERENCE TO RELATED APPLICATIONS

This patent application claims the benefit of U.S. Provisional Patent Applications Nos. 61/313,050, filed March 11, 2010, and 61/313,047, also filed March 11, 2010, both of which are incorporated by reference in their entireties.

### BACKGROUND OF THE INVENTION

Secreted Protein, Acidic, Rich in Cysteines (SPARC), also known as osteonectin, is a 286 amino acid glycoprotein. SPARC has affinity for a wide variety of ligands including cations (e.g., Ca²⁺, Cu²⁺, Fe²⁺), growth factors (e.g., platelet derived growth factor (PDGF), and vascular endothelial growth factor (VEGF)), extracellular matrix (ECM) proteins (e.g., collagen I-V and collagen IX, vitronectin, and thrombospondin-1), endothelial cells, platelets, albumin, and hydroxyapaptite. SPARC expression is developmentally regulated, and is predominantly expressed in tissues undergoing remodeling during normal development or in response to injury (see, e.g., Lane et al., FASEB J., 8, 163-173 (1994)). High levels of SPARC protein are expressed in developing bones and teeth.

SPARC is upregulated in several aggressive cancers, but is absent from the vast majority of normal tissues (Porter et al., J. Histochem. Cytochem., 43, 791(1995) and see below). SPARC expression is induced among a variety of tumors (e.g., bladder, liver, ovary, kidney, gut, and breast). In bladder cancer, for example, SPARC expression has been associated with advanced carcinoma. Invasive bladder tumors of stage T2 or greater have been shown to express higher levels of SPARC than bladder tumors of stage T1 (or less superficial tumors), and have poorer prognosis (see, e.g., Yamanaka et al., J. Urology, 166, 2495-2499 (2001)). In meningiomas, SPARC expression has been associated with invasive tumors only (see, e.g., Rempel et al., Clincal Cancer Res., 5, 237-241 (1999)). SPARC expression also has been detected in 74.5 % of *in situ* invasive breast carcinoma lesions (see, e.g., Bellahcene, et al., Am. J. Pathol., 146, 95-100 (1995)), and 54.2% of infiltrating ductal carcinoma of the breast (see, e.g., Kim et al., J. Korean Med. Sci., 13, 652-657 (1998)). SPARC expression also has been associated with frequent microcalcification in breast cancer (see, e.g., Bellahcene et al., *supra*), suggesting that SPARC expression may be responsible for the affinity of breast metastases for the bone. SPARC is also known to bind albumin (see, e.g., Schnitzer, J. Biol. Chem., 269, 6072 (1994)).

Accordingly, there is a need for compositions and methods that take advantage of SPARC's role in disease, e.g., SPARC's role in some cancers. In particular, there is a need for compositions and methods that take advantage of SPARC's domain specific activities, such as the SPARC carboxy angiogenic domain.

### BRIEF SUMMARY OF THE INVENTION

The invention provides isolated "SPARC angiogenic domain polypeptides," uses of said polypeptides, and methods of detecting and quantifying said polypeptides. Further, the invention provides uses of the "SPARC angiogenic domain" and methods of detecting and quantifying polypeptides comprising this domain.

The invention provides methods of treating an animal suffering from a SPARC-dependent disease with a therapy comprising: (a) quantifying the amount of SPARC angiogenic domain polypeptides and, optionally, full length SPARC comprising the SPARC angiogenic domain at a disease site in said animal, (b) quantifying the amount of SPARC angiogenic domain polypeptides and, optionally, full length SPARC comprising the SPARC angiogenic domain at a disease site in one or more other animals suffering from the same SPARC dependent condition or disease who are known to respond to the therapy, (c) calculating the average of the amounts of SPARC angiogenic domain polypeptides and, if included, full length SPARC comprising the SPARC angiogenic domain determined in (b); (d) comparing said amount determined in (a) to said average determined in (c), and (e) administering the therapy if the amount determined in (a) is greater than or equal to the average determined in (c).

By a "SPARC-dependent disease" it is meant a disease or condition which requires a certain level of SPARC for its maintenance. By "one or more other animals suffering from a SPARC dependent condition or disease who are known to respond to the therapy," it meant at least one animal, but also includes any number that leads to statistically significant comparison with the animal in (a).

The SPARC angiogenic domain and SPARC angiogenic domain polypeptides present in disease-site biopsy material may be detected and quantified by any suitable means known to those of ordinary skill, including, e.g., antibodies directed to the SPARC angiogenic domain used in conventional immunohistologic and solution based assays (e.g., ELISA) and mass spectroscopy. By "disease site" it is meant an anatomical location where the disease is active.

The invention provides isolated polypeptides which comprises a full length SPARC polypeptide lacking the consecutive amino acids of SEQ ID NO: 1 and isolated, carboxy truncated, SPARC polypeptides which are the product of an enzymatic digestion of the carboxy terminus of a full length SPARC polypeptide and which retains no more than 5% of the angiogenic activity SEQ ID NO: 1.

The invention provides methods of treating a tumor in an animal comprising the administration of a therapeutically effective amount of any one or more of the SPARC polypeptides lacking angiogenic activity, including, e.g. isolated, carboxy truncated, SPARC polypeptides which are the product of an enzymatic digestion of the carboxy terminus of a full length SPARC polypeptide and which retains no more than 5% of the angiogenic activity SEQ ID NO: 1 disclosed herein, in particular SEQ ID NO: 2.

The invention provides methods of predicting a positive response of an animal suffering from a SPARC-dependent disease to a therapy comprising: (a) quantifying the amount of SPARC angiogenic domain polypeptides and full length SPARC polypeptides comprising the SPARC angiogenic domain at the disease site, (b) comparing said amount of SPARC to the amount of SPARC present in animals suffering from a SPARC dependent disease who are known to respond to the therapy, and (c) predicting a positive response to the therapy if amount of polypeptides comprising SEQ ID NO: 1 are above or below a threshold level.

The invention provides methods of treating an animal with a SPARC dependent condition or disease comprising inoculating the animal with an immunologically effective amount (i.e., an amount that elicits an immune response) of an immunogen comprising a SPARC angiogenic domain polypeptide.

The invention provides methods of treating an animal with a SPARC dependent condition or disease comprising administering to the animal a therapeutically effective amount a polypeptide which binds to a SPARC angiogenic domain, inhibits the activity of the SPARC angiogenic domain , and concentrates at a disease site. Suitable SPARC angiogenic domain binding peptides include, e.g., wherein polypeptide is conjugated to a chemotherapeutic, radiation or biologic agent. Suitable SPARC angiogenic domain binding polypeptides include an antibodies and antibody fragments.

### BRIEF DESCRIPTION OF THE DRAWINGS

FIG. 1 depicts a graphical representation of data demonstrating the effect of exogenous SPARC administration with Abraxane and Sutent in a PC3 model.

FIG. 2 depicts results from a HUVEC 3-D tube formation assay characterizing the angiogenic activity of SPARC.

FIG. 3 depicts the results of an SDS-PAGE assay in which wildtype SPARC was run alongside SPARC-d (a mixture of two C-terminal truncated SPARC proteins).

FIG. 4 depicts a graphical representation of data obtained from HUVEC 3-D tube formation assay characterizing wildtype SPARC and SPARC-d.

### DETAILED DESCRIPTION OF THE INVENTION

The human SPARC gene encodes a 303 amino acid SPARC protein (SEQ ID NO: 1). This primary translation product is processed by cleavage of the amino terminal signal sequence resulting in the mature form of SPARC which is a 285 amino acid glycoprotein (collectively forms of "full length SPARC"). After cleavage of the amino terminal signal sequence a 32-kD secreted form is produced which migrates at 43 kD on SDS-PAGE because of glycosylation. Crystallographic data indicates the SPARC protein has three modular domains. The N-terminal domain is acidic and binds calcium. The central "follistatin-like domain" contains amino acids involved in the inhibition of angiogenesis and focal adhesion plaque formation and the (K)GHK angiogenic peptide. The carboxyl terminal "E-C domain" contains the amino acids involved in high affinity calcium binding and the inhibition of cell spreading (Yan & Sage, J. Histochem. Cytochem. 7:1495-1505, 1999).

Surprisingly, it has been determined that SPARC polypeptide's pro-angiogenic activity is localized to amino acids 233-286 (SEQ ID NO: 1) of a mature SPARC polypeptide. Previously this activity was reported to be in the N-terminus region of SPARC (Sage H. Adv Dent Res. 1995 9(3 Suppl):5. Since the proapoptotic region of SPARC is more amino terminal, this discovery suggests that SPARC polypeptides lacking the carboxy terminal angiogenesis domain (e.g., SEQ ID NO: 2) may be more active against SPARC dependent diseases, such as, e.g., tumors than full length mature SPARC polypeptides. Additionally, while not desiring to be bound by any particular theory, since angiogenesis is necessary for tumor growth, it is possible that SPARC without the C- terminal angiogenic domain (SEQ ID NO: 2) may compete against full length wild type SPARC and negate its pro-angiogenic, pro-tumor activity in vivo.

The invention provides for uses of the "SPARC angiogenic domain." As used herein this term includes SEQ ID NO: 1 and related sequences, e.g., embodiments wherein SEQ ID NO: 1 has up to 5 conservative amino acid changes, preferably up to 4 conservative amino acid changes, more preferably up to 3 conservative amino acid changes; more preferably up to 2 conservative amino acid changes, more preferably a single conservative amino acid change and which retain at least 60%, preferably at least 50%, more preferably at least 40% , and most preferably at least 30% of the angiogenic activity SEQ ID NO: 1.

As used herein "SPARC angiogenic domain" also includes SEQ ID NO: 1 with up to 5 nonconservative amino acid changes, preferably up to 4 nonconservative amino acid changes, more preferably up to 3 nonconservative amino acid changes; more preferably up to 2 nonconservative amino acid changes, more preferably a single nonconservative amino acid change and which retain at least 60%, preferably at least 50%, more preferably at least 40% , and most preferably at least 30% of the angiogenic activity SEQ ID NO: 1.

As used herein "SPARC angiogenic domain" also includes sequences that are at least 90% identical to SEQ ID NO: 1, preferably that are at least 85% identical to SEQ ID NO: 1, more preferably that are at least 80% identical to SEQ ID NO: 1, more preferably that are at least 75% identical to SEQ ID NO: 1, more preferably that are at least 70% identical to SEQ ID NO: 1 and which retain at least 60%, preferably at least 50%, more preferably at least 40% , and most preferably at least 30% of the angiogenic activity SEQ ID NO: 1. The per cent identity is based on an alignment by any suitable computer program known to those of ordinary skill in the art, including e.g., ClustalW, MAFFT or mAlign or as discussed below.

The invention provides isolated polypeptides of SEQ ID NO: 1 ("SPARC angiogenic domain polypeptides") or 2 with up to an additional 15 amino acids, preferably up to an additional 12 amino acids, more preferably up to an additional 10 amino acids, more preferably up to an additional 8 amino acids, more preferably up to an additional 5 amino acids, more preferably up to an additional 4 amino acids, more preferably up to an additional 3 amino acids, more preferably up to an additional 2, more amino acids, and most preferably an additional amino acid added to the carboxy and/or amino termini. Accordingly, the invention limits the size of the "SPARC angiogenic domain polypeptides," as the term is used herein.

The invention provides isolated "SPARC angiogenic domain polypeptides" including, e.g., polypeptides of SEQ ID NO: 1 with no or up to 5 conservative amino acid changes, preferably up to 4 conservative amino acid changes, more preferably up to 3 conservative amino acid changes; more preferably up to 2 conservative amino acid changes, more preferably a single conservative amino acid change and which retain at least 60%, preferably at least 50%, more preferably at least 40% , and most preferably at least 30% of the angiogenic activity SEQ ID NO: 1.

The invention also provides isolated isolated "SPARC angiogenic domain polypeptides" including, e.g., polypeptides of SEQ ID NO: 1 with no or up to 5 nonconservative amino acid changes, preferably up to 4 nonconservative amino acid changes, more preferably up to 3 nonconservative amino acid changes; more preferably up to 2 nonconservative amino acid changes, more preferably a single nonconservative amino acid change and which retain at least 60%, preferably at least 50%, more preferably at least 40%, and most preferably at least 30% of the angiogenic activity SEQ ID NO: 1.

The invention provides isolated isolated SPARC angiogenic domain polypeptides including, e.g., polypeptides that are at least 90% identical to SEQ ID NO: 1, preferably that are at least 85% identical to SEQ ID NO: 1, more preferably that are at least 80% identical to SEQ ID NO: 1, more preferably that are at least 75% identical to SEQ ID NO: 1, more preferably that are at least 70% identical to SEQ ID NO: 1 and which retain at least 60%, preferably at least 50%, more preferably at least 40% , and most preferably at least 30% of the angiogenic activity SEQ ID NO: 1. The per cent identity is based on an alignment by any suitable computer program known to those of ordinary skill in the art, including e.g., ClustalW, MAFFT or mAlign or as discussed below.

The invention includes isolated polynucleotides comprising a nucleic acid sequence encoding any one of the SPARC polypeptides of the invention described herein, including SEQ ID NOS: 1 and 2 and their mutants disclosed herein, expression vector for expressing such nucleic acid sequences and transformed cells comprising such polynucleotides.

The invention provides methods for stimulating angiogenesis in an animal in need of angiogenesis comprising administering a therapeutically effective amount of isolated isolated SPARC angiogenic domain polypeptides including, e.g., polynucleotides encoding polypeptides comprising the sequence of SEQ ID NO: 1. The invention provides methods for treating and preventing SPARC dependent diseases in an animal comprising administering a therapeutically effective amount of isolated polynucleotides encoding polypeptides comprising the sequence of SEQ ID NO: 2.

The invention provides methods for stimulating angiogenesis in an animal in need of angiogenesis comprising administering a therapeutically effective amount of a purified isolated SPARC angiogenic domain polypeptides including, e.g., polypeptide comprising the sequence of SEQ ID NO: 1 or a mutant thereof which is in accordance with the invention and/or described herein. Accordingly, the invention provides methods of treating pathological hypoperfusion such as restenosis, atherosclerosis, and limbic hypoperfusion; also for ischemia including, e.g., wherein the ischemia is cardiac ischemia and stroke.

The invention provides isolated isolated SPARC polypeptides lacking the angiogenic domain including, e.g., SPARC polypeptides comprising SEQ ID NO: 2., i.e., mature SPARC polypeptides lacking the consecutive amino acids of SEQ ID NO: 1. The invention provides isolated SPARC polypeptides, including epitope tagged polypeptides, which are carboxy truncated, i.e., SPARC polypeptides which are the products of an enzymatic digestion of the carboxy terminus of a full length SPARC polypeptide and which retains no more than 5% of the angiogenic activity SEQ ID NO: 1, preferably no more than 3% of the angiogenic activity SEQ ID NO: 1, more preferably no more than 1% of the angiogenic activity SEQ ID NO: 1, most preferably no more than 1% of the angiogenic activity SEQ ID NO: 1.

Carboxyl digestion can be done by any suitable method including enzymatic and chemical digestions. For example, those of ordinary skill could routinely adapt serine carboxypeptidase, lysosomal Pro-X carboxypeptidase, carboxypeptidase c, carboxypeptidase D, Cysteine type carboxypeptidases, metalloexopeptidases and the like for this purpose. See also, Nakazawa T et al. Terminal proteomics: N- and C-terminal analyses for high-fidelity identification of proteins using MS., Proteomics. 2008 Feb;8(4):673-85 which is hereby incorporated by reference. The invention provides isolated polypeptides of SEQ ID NO: 2 with up to 5 conservative amino acid changes, preferably up to 4 conservative amino acid changes, more preferably up to 3 conservative amino acid changes; more preferably up to 2 conservative amino acid changes, more preferably a single conservative amino acid change and which retains no more than 5% of the angiogenic activity SEQ ID NO: 1, preferably no more than 3% of the angiogenic activity SEQ ID NO: 1, more preferably no more than 1% of the angiogenic activity SEQ ID NO: 1, most preferably no more than 1% of the angiogenic activity SEQ ID NO: 1.

The invention also provides isolated polypeptides of SEQ ID NO: 2 with up to 5 nonconservative amino acid changes, preferably up to 4 nonconservative amino acid changes, more preferably up to 3 nonconservative amino acid changes; more preferably up to 2 nonconservative amino acid changes, more preferably a single nonconservative amino acid change and which retains no more than 5% of the angiogenic activity SEQ ID NO: 1, preferably no more than 3% of the angiogenic activity SEQ ID NO: 1, more preferably no more than 1% of the angiogenic activity SEQ ID NO: 1, most preferably no more than 1% of the angiogenic activity SEQ ID NO: 1.

The invention provides isolated polypeptides that are at least 90% identical to SEQ ID NO: 2, preferably that are at least 85% identical to SEQ ID NO: 2, more preferably that are at least 80% identical to SEQ ID NO: 2, more preferably that are at least 75% identical to SEQ ID NO: 2, more preferably that are at least 70% identical to SEQ ID NO: 2 and which retains no more than 5% of the angiogenic activity SEQ ID NO: 1, preferably no more than 3% of the angiogenic activity SEQ ID NO: 1, more preferably no more than 1% of the angiogenic activity SEQ ID NO: 1, most preferably no more than 1% of the angiogenic activity SEQ ID NO: 1. The per cent identity is based on an alignment by any suitable computer program known to those of ordinary skill in the art, including e.g., ClustalW, MAFFT or mAlign or as discussed below.

The invention provides methods of treating a tumor in an animal comprising the administration of a therapeutically effective amount of any one or more isolated polypeptides of SEQ ID NO: 2 with up to 5 conservative amino acid changes, preferably up to 4 conservative amino acid changes, more preferably up to 3 conservative amino acid changes; more preferably up to 2 conservative amino acid changes, more preferably a single conservative amino acid change and which retains no more than 5% of the angiogenic activity SEQ ID NO: 1, preferably no more than 3% of the angiogenic activity SEQ ID NO: 1, more preferably no more than 1% of the angiogenic activity SEQ ID NO: 1, most preferably no more than 1% of the angiogenic activity SEQ ID NO: 1.

The invention provides methods of treating a tumor in an animal comprising the administration of a therapeutically effective amount of any one or more isolated polypeptides of SEQ ID NO: 2 with up to 5 nonconservative amino acid changes, preferably up to 4 nonconservative amino acid changes, more preferably up to 3 nonconservative amino acid changes; more preferably up to 2 nonconservative amino acid changes, more preferably a single nonconservative amino acid change and which retains no more than 5% of the angiogenic activity SEQ ID NO: 1, preferably no more than 3% of the angiogenic activity SEQ ID NO: 1, more preferably no more than 1 % of the angiogenic activity SEQ ID NO: 1, most preferably no more than 1% of the angiogenic activity SEQ ID NO: 1.

The invention provides methods of sensitizing a tumor in an animal comprising the administration of a therapeutically effective amount any one or more isolated polypeptides of SEQ ID NO: 2 with up to 5 conservative amino acid changes, preferably up to 4 conservative amino acid changes, more preferably up to 3 conservative amino acid changes; more preferably up to 2 conservative amino acid changes, more preferably a single conservative amino acid change and which retains no more than 5% of the angiogenic activity SEQ ID NO: 1, preferably no more than 3% of the angiogenic activity SEQ ID NO: 1, more preferably no more than 1% of the angiogenic activity SEQ ID NO: 1, most preferably no more than 1% of the angiogenic activity SEQ ID NO: 1 and a non-SPARC therapy.

The invention provides methods of sensitizing a tumor in an animal comprising the administration of a therapeutically effective amount any one or more isolated polypeptides of SEQ ID NO: 2 with up to 5 nonconservative amino acid changes, preferably up to 4 nonconservative amino acid changes, more preferably up to 3 nonconservative amino acid changes; more preferably up to 2 nonconservative amino acid changes, more preferably a single nonconservative amino acid change and which retains no more than 5% of the angiogenic activity SEQ ID NO: 1, preferably no more than 3% of the angiogenic activity SEQ ID NO: 1, more preferably no more than 1% of the angiogenic activity SEQ ID NO: 1, most preferably no more than 1% of the angiogenic activity SEQ ID NO: 1 and a non-SPARC therapy.

As used herein, a "SPARC-dependent disease" refers to a disease or condition, e.g, a disease that requires an adequate amount of wild-type SPARC to sustain it pathogenic process. As used herein, an "anti-SPARC therapy" is a therapy comprising molecules that diminish SPARC's activity or target SPARC so as to inactivate it. For Example an anti-SPARC therapies can be a SPARC-binding molecule that delivers an active agent to the site of a tumor or other disease and an antibody directed to SPARC. SPARC dependent diseases and conditions include, e.g., tumors, arthropathies, glomerulonephrities, cholangitis, exaggerated wound healing, remodeling, or angiogenesis.

The invention also provides for the treatment or sensitization of proliferative diseases other than tumors or cancer with therapeutically effective amount any one or more isolated polypeptides of SEQ ID NO: 2 or mutants thereof described herein. Proliferative diseases suitable for treatment hypertrophic scars and keloids, proliferative diabetic retinopathy, rheumatoid arthritis, arteriovenous malformations, atherosclerotic plaques, delayed wound healing, hemophilic joints, nonunion fractures, Osler-Weber syndrome, psoriasis, pyogenic granuloma, scleroderma, tracoma, menorrhagia, vascular adhesions and restenosis.

The invention provides methods of treating or sensitizing a tumor in an animal, wherein the tumor is selected from the group consisting of oral cavity tumors, pharyngeal tumors, digestive system tumors, respiratory system tumors, bone tumors, cartilaginous tumors, bone metastases, sarcomas, skin tumors, melanoma, breast tumors, genital system tumors, urinary tract tumors, orbital tumors, brain and central nervous system tumors, gliomas, endocrine system tumors, thyroid tumors, esophageal tumors, gastric tumors, small intestinal tumors, colonic tumors, rectal tumors, anal tumors, liver tumors, gall bladder tumors, pancreatic tumors, laryngeal tumors, tumors of the lung, bronchial tumors, non-small cell lung carcinoma, small cell lung carcinoma, uterine cervical tumors, uterine corpus tumors, ovarian tumors, vulvar tumors, vaginal tumors, prostate tumors, prostatic carcinoma, testicular tumors, tumors of the penis, urinary bladder tumors, tumors of the kidney, tumors of the renal pelvis, tumors of the ureter, head and neck tumors, parathyroid cancer, Hodgkin's disease, Non-Hodgkin's lymphoma, multiple myeloma, leukemia, acute lymphocytic leukemia, chronic lymphocytic leukemia, acute myeloid leukemia, chronic myeloid leukemia.

The invention provides methods of sensitizing a tumor in an animal, wherein the a non-SPARC therapy is one or more of a chemotherapeutic, radiation or biologic regimen, including e.g., wherein the non-SPARC therapy comprises one or more of docetaxel, paclitaxel, taxanes, platinum compounds, antifolates, antimetabolites, antimitotics, DNA damaging agents, proapoptotics, differentiation inducing agents, antiangiogenic agents, antibiotics, hormones, peptides, antibodies, and combinations thereof.

The invention provides methods of identifying an angiogenesis inhibitor comprising: (a) administering an effective amount of a composition of any one of SEQ ID NO: 1 or mutants thereof to an angiogenesis model system; (b) separately simultaneously administering a candidate angiogenesis inhibitor and the composition of any one of claims 1-4 to the angiogenesis model system; (c) quantifying the amount of angiogenesis produced in (a) and (b); and (d) if angiogenesis is reduced in (b) in comparison to (a), identifying the candidate angiogenesis inhibitor as an actual angiogenesis inhibitor. Any suitable angiogenic model system may be used in accordance with the invention, including e.g., wherein the angiogenesis model system is the HUVEC tube formation assay.

As used herein, a "medicament" is a composition capable of producing an effect that may be administered to a patient or test subject. The effect may be chemical, biological or physical, and the patient or test subject may be human, or a non-human animal, such as a rodent or transgenic mouse. The composition may include small organic or inorganic molecules with distinct molecular composition made synthetically, found in nature, or of partial synthetic origin. Included in this group are nucleotides, nucleic acids, amino acids, peptides, polypeptides, proteins, peptide nucleic acids or complexes comprising at least one of these entities. The medicament may be comprised of the effective composition alone or in combination with a pharmaceutically acceptable excipient.

As used herein, a "pharmaceutically acceptable excipient" includes any and all solvents, dispersion media, coatings, antibacterial, antimicrobial or antifungal agents, isotonic and absorption delaying agents, and the like that are physiologically compatible. The excipient may be suitable for intravenous, intraperitoneal, intramuscular, intrathecal or oral administration. The excipient may include sterile aqueous solutions or dispersions for extemporaneous preparation of sterile injectable solutions or dispersion. Use of such media for preparation of medicaments is known in the art.

As used herein, a "pharmacologically effective amount" or "effective amount" of a medicament refers to using an amount of a medicament present in such a concentration to result in a therapeutic level of drug delivered over the term that the drug is used. This may be dependent on the mode of delivery, time period of the dosage, age, weight, general health, sex and diet of the subject receiving the medicament. The determination of what dose is a "pharmacologically effective amount" requires routine optimization, which is within the capabilities of one of ordinary skill in the art.

As used herein, the terms "cancer" or "tumor" refers to a proliferative disorder caused or characterized by the proliferation of cells which have lost susceptibility to normal growth control. The term cancer, as used in the present application, includes tumors and any other proliferative disorders. Cancers of the same tissue type usually originate in the same tissue, and may be divided into different subtypes based on their biological characteristics. Four general categories of cancers are carcinoma (epithelial tissue derived), sarcoma (connective tissue or mesodermal derived), leukemia (blood-forming tissue derived) and lymphoma (lymph tissue derived). Over 200 different types of cancers are known, and every organ and tissue of the body may be affected. Specific examples of cancers that do not limit the definition of cancer may include melanoma, leukemia, astrocytoma, glioblastoma, retinoblastoma, lymphoma, glioma, Hodgkins' lymphoma and chronic lymphocyte leukemia. Examples of organs and tissues that may be affected by various cancers include pancreas, breast, thyroid, ovary, uterus, testis, prostate, thyroid, pituitary gland, adrenal gland, kidney, stomach, esophagus, colon or rectum, head and neck, bone, nervous system, skin, blood, nasopharyngeal tissue, lung, urinary tract, cervix, vagina, exocrine glands and endocrine glands. Alternatively, a cancer may be multicentric or of unknown primary site (CUPS).

As used herein, a "cancerous cell" refers to a cell that has undergone a transformation event and whose growth is no longer regulated to the same extent as before said transformation event. A tumor refers to a collection of cancerous cells, often found as a solid or semi-solid lump in or on the tissue or a patient or test subject.

Diseases or conditions with pathologic hypoperfusion may be treated in accordance with the invention wherein effective amounts of one or more polypeptides comprising SEQ ID NO: 1 are administered to the animal, such as human. Suitable hypoprofusion diseases or conditions for treatment in accordance with the invention include: cardiac ischemia, myocardial infarction, diabetes, neuropathies, ALS, oral ulcers, gastric ulcers, restenosis, stroke, TIAs, pre-eclampsia and the like (See also, Carmeliet, Angiogenesis in health and disease, Nature Medicine 9, 653 - 660 (2003), which is hereby incorporated by reference, for additional suitable diseases and conditions.)

Diseases with exaggerated angiogenesis, particularly if SPARC-dependent, may be treated in accordance with the invention wherein effective amounts of, e.g., one or more antibodies targeting the SPARC angiogenic domain or other anti-SPARC therapy are administered to the animal, such as a human. Suitable diseases with exaggerated angiogenesis for treatment in accordance with the invention include: cancer, tumors, angioma, endometriosis, diabetic retinopathy, retinopathy of prematurity, psoriasis, arthritis, pyogenic granuloma, angioimmunoblastic lymphadenopathy, periodontal disease, and the like (See also, Carmeliet, Angiogenesis in health and disease, Nature Medicine 9, 653 - 660 (2003), which is hereby incorporated by reference, for additional suitable diseases and conditions.)

Diseases with exaggerated wound healing and remodeling particularly if SPARC-dependent, may be treated in accordance with the invention wherein effective amounts of one or more antibodies targeting the SPARC angiogenic domain or other anti-SPARC therapy are administered to the animal, such as a human. Suitable diseases with exaggerated wound healing and remodeling for treatment in accordance with the invention include: keloids, hyperthrophic scars, pulmonary fibrosis, and and the like (See also, Carmeliet, Angiogenesis in health and disease, Nature Medicine 9, 653 - 660 (2003), which is hereby incorporated by reference, for additional suitable diseases and conditions.)

A cancer or cancerous cell may be described as "sensitive to" or "resistant to" a given therapeutic regimen or chemotherapeutic agent based on the ability of the regimen to kill cancer cells or decrease tumor size, reduce overall cancer growth (i.e. through reduction of angiogenesis), and/or inhibit metastasis. Cancer cells that are resistant to a therapeutic regimen may not respond to the regimen and may continue to proliferate. Cancer cells that are sensitive to a therapeutic regimen may respond to the regimen resulting in cell death, a reduction in tumor size, reduced overall growth (tumor burden) or inhibition of metastasis. For example, this desirably manifest itself in a reduction in tumor size, overall growth/tumor burden or the incidence of metastasis of about 10% or more, for example, about 30%, about 40%, about 50%, about 60%, about 70%, about 80%, or more, to about 2-fold, about 3-fold, about 4-fold, about 5-fold, about 10-fold, about 15-fold, about 20-fold or more. Monitoring of a response may be accomplished by numerous pathological, clinical and imaging methods as described herein and known to persons of skill in the art.

A common theme for a chemotherapeutic agent or combination of agents is to induce death of the cancerous cells. For example, DNA adducts such as nitrosoureas, busulfan, thiotepa, chlorambucil, cisplatin, mitomycin, procarbazine, or dacacarbazine slow the growth of the cancerous cell by forcing the replicating cell to repair the damaged DNA before the M-phase of the cell cycle, or may by themselves cause sufficient damage to trigger apoptosis of the cancerous cell. Other events such as gene expression or transcription, protein translation, or methylation of the replicated DNA, for example, may also be interfered with by the varied arsenal of chemotherapeutic agents available to the clinician and help to trigger apoptotic processes within the cancerous cells. Alternately, a chemotherapeutic agent may enable the cancerous cell to be killed by aspects of the patient or test subject's humoral or acquired immune system, for example, the complement cascade or lymphocyte attack.

While not desiring to be bound by any specific theories, a cancerous cell resistant to a chemotherapeutic agent or combination of agents may fight for its survival by actively transporting the drug out of the cell for example, by overexpression of the ABC transporter MDR1 p-glycoprotein (FORD et al 1993. Cytotechnol. 12:171-212) or acquiring 'counter-mutations' to counteract the drugs. For example, mutations in the DNA repair enzymes that affect the ability to detect damage to the cells' DNA may enable replication of the damaged DNA and permit the cancerous cells to continue replicating, enlarging the tumor. As mutations accumulate, other regulatory points that would otherwise act in a normal cell cycle cease to function, and the cycle of unregulated growth cascades. Another aspect of chemotherapeutic resistance involves the tumor cells' avoidance of apoptosis. A host organism's normal response to dysregulated cell growth is to initiate apoptosis and eliminate the defective cell before the cascade into uncontrolled replication begins. However, this may be subverted by a cancerous cell, for example, by disruption of signal transduction events, loss of adhesion dependence or contact inhibition in the cancerous cell, or loss of apoptosis-promoting factors, often considered 'tumor suppressors', for example p53, BRCA1 or RB. The importance of this sensitivity to apoptosis in the treatment of cancer is supported by recent evidence indicating that the selectivity of chemotherapy for the relatively few tumors ever cured solely by drugs depends, to a large extent, upon their easy susceptibility to undergo apoptosis (Johnstone et al., 2002. Cell. 108(2):153-64).

As used herein, a "therapeutic regimen" or "therapy" refers to the administration of at least one agent which is harmful to cancerous cells. Suitable therapeutic regimens for use in accordance with the invention include, but are not limited to, "chemotherapeutic regimens," "radiotherapeutic regimens," "alternative therapeutic regimen" and combinations thereof.

As used herein, a "chemotherapeutic regimen" or "chemotherapy" refers to the administration of at least one chemotherapy agent which is harmful to destroy cancerous cells. There are a myriad of such chemotherapy agents available to a clinician. Chemotherapy agents may be administered to a subject in a single bolus dose, or may be administered in smaller doses over time. A single chemotherapeutic agent may be used (single-agent therapy) or more than one agent may be used in combination (combination therapy). Chemotherapy may be used alone to treat some types of cancer. Alternatively, chemotherapy may be used in combination with other types of treatment, for example, radiotherapy or alternative therapies (for example immunotherapy) as described herein. Additionally, a chemosensitizer may be administered as a combination therapy with a chemotherapy agent.

As used herein, a "chemotherapeutic agent" refers to a medicament that may be used to treat cancer, and generally has the ability to kill cancerous cells directly. Examples of chemotherapeutic agents include alkylating agents, antimetabolites, natural products, hormones and antagonists, and miscellaneous agents. Examples of alternate names are indicated in brackets. Examples of alkylating agents include nitrogen mustards such as mechlorethamine, cyclophosphamide, ifosfamide, melphalan (L-sarcolysin) and chlorambucil; ethylenimines and methylmelamines such as hexamethylmelamine and thiotepa; alkyl sulfonates such as busulfan; nitrosoureas such as carmustine (BCNU), semustine (methyl-CCNU), lomustine (CCNU) and streptozocin (streptozotocin); DNA synthesis antagonists such as estramustine phosphate; and triazines such as dacarbazine (DTIC, dimethyl-triazenoimidazolecarboxamide) and temozolomide. Examples of antimetabolites include folic acid analogs such as methotrexate (amethopterin); pyrimidine analogs such as fluorouracin (5-fluorouracil, 5-FU, 5FU), floxuridine (fluorodeoxyuridine, FUdR), cytarabine (cytosine arabinoside) and gemcitabine; purine analogs such as mercaptopurine (6-mercaptopurine, 6-MP), thioguanine (6-thioguanine, TG) and pentostatin (2'-deoxycoformycin, deoxycoformycin), cladribine and fludarabine; and topoisomerase inhibitors such as amsacrine. Examples of natural products include vinca alkaloids such as vinblastine (VLB) and vincristine; taxanes such as paclitaxel and docetaxel (Taxotere); epipodophyllotoxins such as etoposide and teniposide; camptothecins such as topotecan and irinotecan; antibiotics such as dactinomycin (actinomycin D), daunorubicin (daunomycin, rubidomycin), doxorubicin, bleomycin, mitomycin (mitomycin C), idarubicin, epirubicin; enzymes such as L-asparaginase; and biological response modifiers such as interferon alpha and interlelukin 2. Examples of hormones and antagonists include luteinising releasing hormone agonists such as buserelin; adrenocorticosteroids such as prednisone and related preparations; progestins such as hydroxyprogesterone caproate, medroxyprogesterone acetate and megestrol acetate; estrogens such as diethylstilbestrol and ethinyl estradiol and related preparations; estrogen antagonists such as tamoxifen and anastrozole; androgens such as testosterone propionate and fluoxymesterone and related preparations; androgen antagonists such as flutamide and bicalutamide; and gonadotropin-releasing hormone analogs such as leuprolide. Examples of miscellaneous agents include thalidomide; platinum coordination complexes such as cisplatin (cis-DDP), oxaliplatin and carboplatin; anthracenediones such as mitoxantrone; substituted ureas such as hydroxyurea; methylhydrazine derivatives such as procarbazine (N-methylhydrazine, MIH); adrenocortical suppressants such as mitotane (o,p'-DDD) and aminoglutethimide; RXR agonists such as bexarotene; and tyrosine kinase inhibitors such as imatinib. Alternate names and trade-names of these and additional examples of chemotherapeutic agents, and their methods of use including dosing and administration regimens, will be known to a person versed in the art, and may be found in any suitable reference know to those of ordinary skill. In particular, suitable chemotherapeutic agents for use in accordance with the invention include, without limitation, nanoparticle albumin-bound paclitaxels.

As used herein, the term "radiotherapeutic regimen" or "radiotherapy" refers to the administration of radiation to kill cancerous cells. Radiation interacts with various molecules within the cell, but the primary target, which results in cell death is the deoxyribonucleic acid (DNA). However, radiotherapy often also results in damage to the cellular and nuclear membranes and other organelles. DNA damage usually involves single and double strand breaks in the sugar-phosphate backbone. Furthermore, there can be cross-linking of DNA and proteins, which can disrupt cell function. Depending on the radiation type, the mechanism of DNA damage may vary as does the relative biologic effectiveness. For example, heavy particles (i.e. protons, neutrons) damage DNA directly and have a greater relative biologic effectiveness. Electromagnetic radiation results in indirect ionization acting through short-lived, hydroxyl free radicals produced primarily by the ionization of cellular water. Clinical applications of radiation consist of external beam radiation (from an outside source) and brachytherapy (using a source of radiation implanted or inserted into the patient). External beam radiation consists of X-rays and/or gamma rays, while brachytherapy employs radioactive nuclei that decay and emit alpha particles, or beta particles along with a gamma ray.

Radiotherapy may further be used in combination chemotherapy, with the chemotherapeutic agent acting as a radiosensitizer. The specific choice of radiotherapy suited to an individual patient may be determined by a skilled person at the point of care, taking into consideration the tissue and stage of the cancer.

As used herein, the term "alternative therapeutic regimen" or "alternative therapy" may include for example, biologic response modifiers (including polypeptide-, carbohydrate-, and lipid-biologic response modifiers), toxins, lectins, antiangiogenic agents, receptor tyrosine kinase inhibitors (for example Iressa® (gefitinib), Tarceva® (erlotinib), Erbitux® (cetuximab), imatinib mesilate (Gleevec®), proteosome inhibitors (for example bortezomib, Velcade); VEGFR2 inhibitors such as PTK787 (ZK222584), aurora kinase inhibitors (for example ZM447439); mammalian target of rapamycin (mTOR) inhibitors, cyclooxygenase-2 (COX-2) inhibitors, rapamycin inhibitors (for example sirolimus, Rapamune.TM.); farnesyltransferase inhibitors (for example tipifarnib, Zarnestra); matrix metalloproteinase inhibitors (for example BAY 12-9566; sulfated polysaccharide tecogalan); angiogenesis inhibitors (for example Avastin.TM. (bevacizumab); analogues of fumagillin such as TNP-4; carboxyaminotriazole; BB-94 and BB-2516; thalidomide; interleukin-12; linomide; peptide fragments; and antibodies to vascular growth factors and vascular growth factor receptors); platelet derived growth factor receptor inhibitors, protein kinase C inhibitors, mitogen-activated kinase inhibitors, mitogen-activated protein kinase kinase inhibitors, Rous sarcoma virus transforming oncogene (SRC) inhibitors, histonedeacetylase inhibitors, small hypoxia-inducible factor inhibitors, hedgehog inhibitors, and TGF-.beta. signalling inhibitors. Furthermore, an immunotherapeutic agent would also be considered an alternative therapeutic regimen. Examples include chemokines, chemotaxins, cytokines, interleukins, or tissue factor. Suitable immunotherapeutic agents also include serum or gamma globulin containing preformed antibodies; nonspecific immunostimulating adjuvants; active specific immunotherapy; and adoptive immunotherapy. In addition, alternative therapies may include other biological-based chemical entities such as polynucleotides, including antisense molecules, polypeptides, antibodies, gene therapy vectors and the like. Such alternative therapeutics may be administered alone or in combination, or in combination with other therapeutic regimens described herein. Alternate names and trade-names of these agents used in alternative therapeutic regimens and additional examples of agents used in alternative therapeutic regimens, and their methods of use including dosing and administration regimens, will be known to a physician versed in the art. Furthermore, methods of use of chemotherapeutic agents and other agents used in alternative therapeutic regimens in combination therapies, including dosing and administration regimens, will also be known to a person versed in the art.

In particular, suitable alternative therapeutic regimens include, without limitation, antibodies to molecules on the surface of cancer cells such as antibodies to Her2 (e.g., Trastuzumab), EGF or EGF Receptors, VEGF (e.g., Bevacizumab) or VEGF Receptors, CD20, and the like. The therapeutic agent may further comprise any antibody or antibody fragment which mediates one or more of complement activation, cell mediated cytotoxicity, inducing apoptosis, inducing cell death, and opsinization. For example, such an antibody fragment may be a complete or partial Fc domain.

By "antibodies" it is meant without limitation, monoclonal antibodies, polyclonal antibodies, dimers, multimers, multispecific antibodies (e.g., bispecific antibodies). Antibodies may be murine, human, humanized, chimeric, or derived from other species. An antibody is a protein generated by the immune system that is capable of recognizing and binding to a specific antigen. A target antigen generally has numerous binding sites, also called epitopes, recognized by CDRs on multiple antibodies. Each antibody that specifically binds to a different epitope has a different structure. Thus, one antigen may have more than one corresponding antibody.

An antibody includes a full-length immunoglobulin molecule or an immunologically active portion of a full-length immunoglobulin molecule, i.e., a molecule that contains an antigen binding site that immunospecifically binds an antigen of a target of interest or part thereof. Targets include, cancer cells or other cells that produce autoimmune antibodies associated with an autoimmune disease.

The immunoglobulins disclosed herein can be of any class (e.g., IgG, IgE, IgM, IgD, and IgA) or subclass (e.g., IgG1, IgG2, IgG3, IgG4, IgA1 and IgA2) of immunoglobulin molecule. The immunoglobulins can be derived from any species.

"Antibody fragments" comprise a portion of a full length antibody, which maintain the desired biological activity. "Antibody fragments" are generally the antigen binding or variable region thereof. Examples of antibody fragments include Fab, Fab', F(ab')₂, and Fv fragments; diabodies; linear antibodies; fragments produced by a Fab expression library, anti-idiotypic (anti-Id) antibodies, CDR (complementary determining region), and epitope-binding fragments of any of the above which immunospecifically bind to cancer cell antigens, viral antigens or microbial antigens, single-chain antibody molecules; and multispecific antibodies formed from antibody fragments.

The monoclonal antibodies referenced herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Pat. No. 4,816,567). Chimeric antibodies of interest herein include "primatized" antibodies comprising variable domain antigen-binding sequences derived from a non-human primate (e.g., Old World Monkey or Ape) and human constant region sequences.

Antibody-dependent cell-mediated cytotoxicity" and "ADCC" refer to a cell-mediated reaction in which nonspecific cytotoxic cells that express Fc receptors (FcRs) (e.g., Natural Killer (NK) cells, neutrophils, and macrophages) recognize bound antibody on a target cell and subsequently cause lysis of the target cell. The primary cells for mediating ADCC, NK cells, express Fc..gamma..RIII only, whereas monocytes express Fc.gamma.RI, Fc.gamma.RII and Fc.gamma.RIII. To assess ADCC activity of a molecule of interest, an in vitro ADCC assay may be performed (U.S. Pat. No. 5,003,621; U.S. Pat. No. 5,821,337). Useful effector cells for such assays include peripheral blood mononuclear cells (PBMC) and Natural Killer (NK) cells. Alternatively, or additionally, ADCC activity of the molecule of interest may be assessed in vivo, e.g., in a animal model such as that disclosed in Clynes et al PNAS (USA), 95:652-656 (1998).

An antibody which "induces cell death" is one which causes a viable cell to become nonviable. Cell death in vitro may be determined in the absence of complement and immune effector cells to distinguish cell death induced by antibody-dependent cell-mediated cytotoxicity (ADCC) or complement dependent cytotoxicity (CDC). Thus, the assay for cell death may be performed using heat inactivated serum (i.e., in the absence of complement) and in the absence of immune effector cells. To determine whether the antibody is able to induce cell death, loss of membrane integrity as evaluated by uptake of propidium iodide (PI), trypan blue or 7AAD can be assessed relative to untreated cells. Cell death-inducing antibodies are those which induce PI uptake in the PI uptake assay in BT474 cells.

An antibody which "induces apoptosis" is one which induces programmed cell death as determined by binding of annexin V, fragmentation of DNA, cell shrinkage, dilation of endoplasmic reticulum, cell fragmentation, and/or formation of membrane vesicles (called apoptotic bodies).

As used herein, a "chemosensitizer" or "sensitizer" is a medicament that may enhance the therapeutic effect of a chemotherapeutic agent, radiotherapy treatment or alternative therapeutic regimen, and therefore improve efficacy of such treatment or agent. The sensitivity or resistance of a tumor or cancerous cell to treatment may also be measured in an animal, such as a human or rodent, by, e.g., measuring the tumor size, tumor burden or incidence of metastases over a period of time. For example, about 2, about 3, about 4 or about 6 months for a human and about 2-4, about 3-5, or about 4-6 weeks for a mouse. A composition or a method of treatment may sensitize a tumor or cancerous cell's response to a therapeutic treatment if the increase in treatment sensitivity or the reduction in resistance is about 10% or more, for example, about 30%, about 40%, about 50%, about 60%, about 70%, about 80%, or more, to about 2-fold, about 3-fold, about 4-fold, about 5-fold, about 10-fold, about 15-fold, about 20-fold or more, compared to treatment sensitivity or resistance in the absence of such composition or method. The determination of sensitivity or resistance to a therapeutic treatment is routine in the art and within the skill of a person versed in the art.

The terms "peptide," "polypeptide," and "protein" may be used interchangeably, and refer to a compound comprised of at least two amino acid residues covalently linked by peptide bonds or modified peptide bonds, for example peptide isosteres (modified peptide bonds) that may provide additional desired properties to the peptide, such as increased half-life. A peptide may comprise at least two amino acids. The amino acids comprising a peptide or protein described herein may also be modified either by natural processes, such as posttranslational processing, or by chemical modification techniques which are well known in the art. Modifications can occur anywhere in a peptide, including the peptide backbone, the amino acid side-chains and the amino or carboxyl termini. It is understood that the same type of modification may be present in the same or varying degrees at several sites in a given peptide.

Examples of modifications to peptides may include PEGylation, acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent crosslinks, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. See, for instance, Proteins-Structure and Molecular Properties, 2.sup.nd ed., T. E. Creighton, W H. Freeman and Company, New York, 1993 and Wold F, Posttranslational Protein Modifications: Perspectives and Prospects, pgs. 1-12 in Posttranslational Covalent Modification of Proteins, B. C. Johnson, ed., Academic Press, New York, 1983; Seifter et al., Analysis for protein modifications and nonprotein cofactors, Meth. Enzymol. (1990) 182: 626-646 and Rattan et al. (1992), Protein Synthesis: Posttranslational Modifications and Aging," Ann NY Acad Sci 663: 48-62.

A substantially similar sequence is an amino acid sequence that differs from a reference sequence only by one or more conservative substitutions as discussed herein. Such a sequence may, for example, be functionally homologous to another substantially similar sequence. It will be appreciated by a person of skill in the art the aspects of the individual amino acids in a peptide of the invention that may be substituted.

Amino acid sequence similarity or identity may be computed by, e.g., using the BLASTP and TBLASTN programs which employ the BLAST (basic local alignment search tool) 2.0 algorithm. Techniques for computing amino acid sequence similarity or identity are well known to those skilled in the art, and the use of the BLAST algorithm is described in Altschul et al. 1990, J Mol. Biol. 215: 403-410 and Altschul et al. (1997), Nucleic Acids Res. 25: 3389-3402.

Sequences on which to perform an alignment may be collected from numerous databases. Examples of protein databases include SWISS-PROT, which also provides a high level of annotation relating to the function of a protein, its domains structure, post-translational modifications, variants (Bairoch A. and Apweiler R. (2000) Nucleic Acids Res. 28(1):45-48; Bairoch A. and Apweiler R. (1997) J. Mol. Med. 75(5):312-316; Junker V. L. et al.(1999) Bioinformatics 15(12):1066-1007), TrEMBL a computer-annotated supplement of SWISS-PROT that contains all the translations of EMBL nucleotide sequence entries (Bairoch A. and Apweiler R. (2000) Nucleic Acids Res. 28(1):45-48) and nr database compares all non-redundant GenBank CDS translations plus protein sequences from other databases such as PDB, SwissProt, PIR and PRF.

Alignments of protein sequences may be conducted using existing algorithms to search databases for sequences similar to a query sequence. One alignment method is the Smith-Waterman algorithm (Smith, T. F. and Waterman, M. S. 1981. Journal of Molecular Biology 147(1):195-197), which is useful in determining how an optimal alignment between the query sequence and a database sequence can be produced. Such an alignment is obtained by determining what transformations the query sequence would need to undergo to match the database sequence. Transformations include substituting one character for another and inserting or deleting a string of characters. A score is assigned for each character-to-character comparison-positive scores for exact matches and some substitutions, negative scores for other substitutions and insertions/deletions. Scores are obtained from statistically-derived scoring matrices. The combination of transformations that results in the highest score is used to generate an alignment between the query sequence and database sequence. The Needleman-Wunsch (Needleman, S. B. and Wunsch, C. D. 1970. Journal of Molecular Biology 48(3):443-453) algorithm is similar to the Smith-Waterman algorithm, but sequence comparisons are global, not local. Global comparisons force an alignment of the entire query sequence against the entire database sequence. While local alignments always begin and end with a match, global alignments may begin or end with an insertion or deletion (indel). For a given query sequence and database sequence, a global score will be less than or equal to a local score due to indels on the ends. As an alternative to the above algorithms, a Hidden Markov Model (HMM) search (Eddy, S. R. 1996. Current Opinion in Structural Biology 6(3):361-365) could be used to generate protein sequence alignments. HMM scoring weighs the probability of a match being followed by insertions/deletions or vice-versa. In addition, HMMs allow insertion to deletion transitions (and vice versa) and scoring of begin and end states to control whether a search is run globally or locally.

One or more of the above algorithms may be used in an alignment program to generate protein sequence alignments. A person skilled in the art has numerous sequence alignment programs to choose from, that incorporate a variety of different algorithms. One example of an alignment program is BLASTP (Altschul, S. F., et al. (1997) Nucleic Acids Res. 25(17):3389-3402). Other alignment programs are CLUSTAL W and PILEUP. The standard output from a BLASTP run contains enough information to conduct further indel analysis as described below.

Amino acids may be described as, for example, polar, non-polar, acidic, basic, aromatic or neutral. A polar amino acid is an amino acid that may interact with water by hydrogen bonding at biological or near-neutral pH. The polarity of an amino acid is an indicator of the degree of hydrogen bonding at biological or near-neutral pH. Examples of polar amino acids include serine, proline, threonine, cysteine, asparagine, glutamine, lysine, histidine, arginine, aspartate, tyrosine and glutamate. Examples of non-polar amino acids include glycine, alanine, valine leucine, isoleucine, methionine, phenylalanine, and tryptophan. Acidic amino acids have a net negative charge at a neutral pH. Examples of acidic amino acids include aspartate and glutamate. Basic amino acids have a net positive charge at a neutral pH. Examples of basic amino acids include arginine, lysine and histidine. Aromatic amino acids are generally nonpolar, and may participate in hydrophobic interactions. Examples of aromatic amino acids include phenylalanine, tyrosine and tryptophan. Tyrosine may also participate in hydrogen bonding through the hydroxyl group on the aromatic side chain. Neutral, aliphatic amino acids are generally nonpolar and hydrophobic. Examples of neutral amino acids include alanine, valine, leucine, isoleucine and methionine. An amino acid may be described by more than one descriptive category. Amino acids sharing a common descriptive category may be substitutable for each other in a peptide.

Nomenclature used to describe the peptide compounds of the present invention follows the conventional practice where the amino group is presented to the left and the carboxy group to the right of each amino acid residue. In the sequences representing selected specific embodiments of the present invention, the amino- and carboxy-terminal groups, although not specifically shown, will be understood to be in the form they would assume at physiologic pH values, unless otherwise specified. In the amino acid structure formulae, each residue may be generally represented by a one-letter or three-letter designation, corresponding to the trivial name of the amino acid.

The hydropathy index of an amino acid is a scale indicating the tendency of an amino acid to seek out an aqueous environment (negative value) or a hydrophobic environment (positive value) (Kyte & Doolittle 1982. J Mol Biol 157:105-132). Hydropathy indices of the standard amino acids include alanine (1.8), arginine (-4.5), asparagine (-3.5), aspartic acid (-3.5), cysteine (2.5), glutamine (-3.5), glutamic acid (-3.5), glycine (-0.4), histidine (-3.2), isoleucine (4.5), leucine (3.8), lysine (-3.9), methionine (1.9), phenylalanine (2.8), proline (-1.6), serine (-0.8), threonine (-0.7), tryptophan (-0.9), tyrosine (-1.3), and valine (4.2). Amino acids with similar hydropathy indices may be substitutable for each other in a peptide.

Amino acids comprising the peptides described herein will be understood to be in the L- or D-configuration. In peptides and peptidomimetics of the present invention, D-amino acids may be substitutable for L-amino acids.

Amino acids contained within the peptides of the present invention, and particularly at the carboxy-or amino-terminus, may be modified by methylation, amidation, acetylation or substitution with other chemical groups which may change the circulating half-life of the peptide without adversely affecting their biological activity. Additionally, a disulfide linkage may be present or absent in the peptides of the invention.

Nonstandard amino acids may occur in nature, and may or may not be genetically encoded. Examples of genetically encoded nonstandard amino acids include selenocysteine, sometimes incorporated into some proteins at a UGA codon, which may normally be a stop codon, or pyrrolysine, sometimes incorporated into some proteins at a UAG codon, which may normally be a stop codon. Some nonstandard amino acids that are not genetically encoded may result from modification of standard amino acids already incorporated in a peptide, or may be metabolic intermediates or precursors, for example. Examples of nonstandard amino acids include 4-hydroxyproline, 5-hydroxylysine, 6-N-methyllysine, gamma-carboxyglutamate, desmosine, selenocysteine, omithine, citrulline, lanthionine, 1-aminocyclopropane-1-carboxylic acid, gamma-aminobutyric acid, carnitine, sarcosine, or N-formylmethionine. Synthetic variants of standard and non-standard amino acids are also known and may include chemically derivatized amino acids, amino acids labeled for identification or tracking, or amino acids with a variety of side groups on the alpha carbon. Examples of such side groups are known in the art and may include aliphatic, single aromatic, polycyclic aromatic, heterocyclic, heteronuclear, amino, alkylamino, carboxyl, carboxamide, carboxyl ester, guanidine, amidine, hydroxyl, alkoxy, mercapto-, alkylmercapto-, or other heteroatom-containing side chains. Other synthetic amino acids may include alpha-imino acids, non-alpha amino acids such as beta-amino acids, des-carboxy or des-amino acids. Synthetic variants of amino acids may be synthesized using general methods known in the art, or may be purchased from commercial suppliers, for example RSP Amino Acids LLC (Shirley, Mass.).

In order to further exemplify what is meant by a conservative amino acid substitution, Groups A-F are listed below. The replacement of one member of the following groups by another member of the same group is considered to be a conservative substitution.

Group A includes leucine, isoleucine, valine, methionine, phenylalanine, serine, cysteine, threonine, and modified amino acids having the following side chains: ethyl, isobutyl, --CH₂CH₂OH, --CH₂CH₂CH₂OH, --CH₂CHOHCH₃ and CH₂SCH₃.

Group B includes glycine, alanine, valine, serine, cysteine, threonine, and a modified amino acid having an ethyl side chain.

Group C includes phenylalanine, phenylglycine, tyrosine, tryptophan, cyclohexylmethyl, and modified amino residues having substituted benzyl or phenyl side chains.

Group D includes glutamic acid, aspartic acid, a substituted or unsubstituted aliphatic, aromatic or benzylic ester of glutamic or aspartic acid (e.g., methyl, ethyl, n-propyl, iso-propyl, cyclohexyl, benzyl, or substituted benzyl), glutamine, asparagine, CO--NH-alkylated glutamine or asparagine (e.g., methyl, ethyl, n-propyl, and iso-propyl), and modified amino acids having the side chain --(CH2)3COOH, an ester thereof (substituted or unsubstituted aliphatic, aromatic, or benzylic ester), an amide thereof, and a substituted or unsubstituted N-alkylated amide thereof.

Group E includes histidine, lysine, arginine, N-nitroarginine, p-cycloarginine, g-hydroxyarginine, N-amidinocitruline, 2-amino guanidinobutanoic acid, homologs of lysine, homologs of arginine, and omithine.

Group F includes serine, threonine, cysteine, and modified amino acids having C1-C5 straight or branched alkyl side chains substituted with --OH or --SH.

Groups A-F are exemplary and are not intended to limit the invention.

A peptidomimetic is a compound comprising non-peptidic structural elements that mimics the biological action of a parent peptide. A peptidomimetic may not have classical peptide characteristics such as an enzymatically scissile peptidic bond. A parent peptide may initially be identified as a binding sequence or phosphorylation site on a protein of interest, or may be a naturally occurring peptide, for example a peptide hormone. Assays to identify peptidomimetics may include a parent peptide as a positive control for comparison purposes, when screening a library, such as a peptidomimetic library. A peptidomimetic library is a library of compounds that may have biological activity similar to that of a parent peptide.

As used herein, the term "polynucleotide" includes RNA, cDNA, genomic DNA, synthetic forms, and mixed polymers, both sense and antisense strands, and may be chemically or biochemically modified or may contain non-natural or derivatized nucleotide bases, as will be readily appreciated by those skilled in the art. Such modifications include, for example, labels, methylation, substitution of one or more of the naturally occurring nucleotides with an analog, internucleotide modifications such as uncharged linkages (e.g., methyl phosphonates, phosphotriesters, phosphoamidates, carbamates, etc.), charged linkages (e.g., phosphorothioates, phosphorodithioates, etc.), pendent moieties (e.g., polypeptides), and modified linkages (e.g., alpha anomeric polynucleotides, etc.). Also included are synthetic molecules that mimic polynucleotides in their ability to bind to a designated sequence via hydrogen bonding and other chemical interactions.

"Peptide nucleic acids" (PNA) as used herein refer to modified nucleic acids in which the sugar phosphate skeleton of a nucleic acid has been converted to an N-(2-aminoethyl)-glycine skeleton. Although the sugar-phosphate skeletons of DNA/RNA are subjected to a negative charge under neutral conditions resulting in electrostatic repulsion between complementary chains, the backbone structure of PNA does not inherently have a charge. Therefore, there is no electrostatic repulsion. Consequently, PNA has a higher ability to form double strands as compared with conventional nucleic acids, and has a high ability to recognize base sequences. Furthermore, PNAs are generally more robust than nucleic acids. PNAs may also be used in arrays and in other hybridization or other reactions as described above and herein for oligonucleotides.

As used herein, the term "vector" refers to a polynucleotide compound used for introducing exogenous or endogenous polynucleotide into host cells. A vector comprises a nucleotide sequence, which may encode one or more polypeptide molecules. Plasmids, cosmids, viruses and bacteriophages, in a natural state or which have undergone recombinant engineering, are non-limiting examples of commonly used vectors to provide recombinant vectors comprising at least one desired isolated polynucleotide molecule.

As used herein, a "tumor suppressor" is a gene or gene product that has a normal biological role of restraining unregulated growth of a cell. If the function of a tumor suppressor is lost, unregulated cell growth arises. The functional counterpart to a tumor suppressor is an oncogene--genes that promote normal cell growth may be known as 'protooncogenes'. A mutation that activates such a gene or gene product further converts it to an 'oncogene', which continues the cell growth activity, but in a dysregulated manner. Examples of tumor suppressor genes and gene products are well known in the literature and may include PTC, BRCA1, BRCA2, p16, APC, RB, WT1, EXT1, p53, NF1, TSC2, NF2, VHL or SPARC.

The invention further provides nucleic acid constructs comprising control elements and a nucleic acid molecule described herein operatively linked to the control elements (e.g., a suitable promoter) for expression of a polypeptide or a polypeptide herein described. Protein expression is dependent on the level of RNA transcription, which is in turn regulated by DNA signals. Similarly, translation of mRNA requires, at the very least, an AUG initiation codon, which is usually located within about 10 to about 100 nucleotides of the 5' end of the message. Sequences flanking the AUG initiator codon have been shown to influence its recognition by eukaryotic ribosomes, with conformity to a perfect Kozak consensus sequence resulting in optimal translation (see, e.g., Kozak, J. Molec. Biol. 196: 947-950 (1987)). Also, successful expression of an exogenous nucleic acid in a cell can require post-translational modification of a resultant protein. Accordingly, the invention provides plasmids encoding polypeptides wherein the vector is, e.g., pCDNA3.1 or a derivative thereof.

The nucleic acid molecules described herein preferably comprise a coding region operatively linked to a suitable promoter, which promoter is preferably functional in eukaryotic cells. Viral promoters, such as, without limitation, the RSV promoter and the adenovirus major late promoter can be used in the invention. Suitable non-viral promoters include, but are not limited to, the phosphoglycerokinase (PGK) promoter and the elongation factor 1.alpha. promoter. Non-viral promoters are desirably human promoters. Additional suitable genetic elements, many of which are known in the art, also can be ligated to, attached to, or inserted into the inventive nucleic acid and constructs to provide additional functions, level of expression, or pattern of expression. The native promoters for expression of the SPARC family genes also can be used, in which event they are preferably not used in the chromosome naturally encoding them unless modified by a process that substantially changes that chromosome. Such substantially changed chromosomes can include chromosomes transfected and altered by a retroviral vector or similar process. Alternatively, such substantially changed chromosomes can comprise an artificial chromosome such as a HAC, YAC, or BAC.

In addition, the nucleic acid molecules described herein may be operatively linked to enhancers to facilitate transcription. Enhancers are cis-acting elements of DNA that stimulate the transcription of adjacent genes. Examples of enhancers which confer a high level of transcription on linked genes in a number of different cell types from many species include, without limitation, the enhancers from SV40 and the RSV-LTR. Such enhancers can be combined with other enhancers which have cell type-specific effects, or any enhancer may be used alone.

To optimize polypeptide production the inventive nucleic acid molecule can further comprise a polyadenylation site following the coding region of the nucleic acid molecule. Also, preferably all the proper transcription signals (and translation signals, where appropriate) will be correctly arranged such that the exogenous nucleic acid will be properly expressed in the cells into which it is introduced. If desired, the exogenous nucleic acid also can incorporate splice sites (i.e., splice acceptor and splice donor sites) to facilitate MRNA production while maintaining an inframe, full length transcript. Moreover, the inventive nucleic acid molecules can further comprise the appropriate sequences for processing, secretion, intracellular localization, and the like.

The nucleic acid molecules can be inserted into any suitable vector. Suitable vectors include, without limitation, viral vectors. Suitable viral vectors include, without limitation, retroviral vectors, alphaviral, vaccinial, adenoviral, adenoassociated viral, herpes viral, and fowl pox viral vectors. The vectors preferably have a native or engineered capacity to transform eukaryotic cells, e.g., CHO-K1 cells. Additionally, the vectors useful in the context of the invention can be "naked" nucleic acid vectors (i.e., vectors having little or no proteins, sugars, and/or lipids encapsulating them) such as plasmids or episomes, or the vectors can be complexed with other molecules. Other molecules that can be suitably combined with the inventive nucleic acids include without limitation viral coats, cationic lipids, liposomes, polyamines, gold particles, and targeting moieties such as ligands, receptors, or antibodies that target cellular molecules.

SPARC polypeptides in accordance the invention can be expressed and purified from a recombinant host cell. Recombinant host cells may be prokaryotic or eukaryotic, including but not limited to bacteria such as E. coli, fungal cells such as yeast, insect cells including but, not limited to, drosophila and silkworm derived cell lines, and mammalian cells and cell lines. When expressing SPARC polypeptides in accordance the invention in a cell, e.g., a human cell, whether, in vitro or in vivo, the codons selected for such the polynucleotide encoding the Q3 SPARC can be optimized for a given cell type (i.e., species). Many techniques for codon optimization are known in the art (see, e.g., Jayaraj et al, Nucleic Acids Res. 33(9):3011-6 (2005); Fuglsang et al., Protein Expr. Purif. 31(2):247-9 (2003); Wu et al., "The Synthetic Gene Designer: a Flexible Web Platform to Explore Sequence Space of Synthetic Genes for Heterologous Expression," csbw, 2005 IEEE Computational Systems Bioinformatics Conference--Workshops (CSBW'05), pp. 258-259 (2005)).

In certain embodiments, when expressing and purifying a SPARC polypeptide, techniques for improving protein solubility are employed to prevent the formation of inclusion body (which are insoluble fractions), and therefore obtaining large quantities of the polypeptide. SPARC accumulated in inclusion bodies is often an inactive-type SPARC not retaining its physiological activities.

Solubility of a purified SPARC polypeptide can be improved by methods known in the art. For example, solubility may also be improved by expressing a functional fragment, but not the full length polypeptide. In addition, to increase the solubility of an expressed protein (e.g., in E. coli), one can reduce the rate of protein synthesis by lowering the growth temperature, using a weaker promoter, using a lower copy number plasmid, lowering the inducer concentration, changing the growth medium as described in Georgiou & Valax (Current Opinion Biotechnol. 7:190-197 (1996)). This decreases the rate of protein synthesis and usually more soluble protein is obtained. One can also add prostethic groups or co-factors which are essential for proper folding or for protein stability, or add buffer to control pH fluctuation in the medium during growth, or add 1% glucose to repress induction of the lac promoter by lactose, which is present in most rich media (such as LB, 2xYT). Polyols (e.g., sorbitol) and sucrose may also be added to the media because the increase in osmotic pressure caused by these additions leads to the accumulation of osmoprotectants in the cell, which stabilize the native protein structure. Ethanol, low molecular weight thiols and disulfides, and NaCl may be added. In addition, chaperones and/or foldases may be co-expressed with the desired polypeptide. Molecular chaperones promote the proper isomerization and cellular targeting by transiently interacting with folding intermediates. E. coli chaperone systems include but, are not limited to: GroES-GroEL, DnaK-DnaJ-GrpE, CIpB.

Foldases accelerate rate-limiting steps along the folding pathway. Three types of foldases play an important role: peptidyl prolyl cis/trans isomerases (PPI's), disulfide oxidoreductase (DsbA) and disulfide isomerase (DsbC), protein disulfide isomerase (PDI) which is an eukaryotic protein that catalyzes both protein cysteine oxidation and disulfide bond isomerization. Co-expression of one or more of these proteins with the target protein could lead to higher levels of soluble target protein.

A SPARC polypeptide can be produced as a fusion protein in order to improve its solubility and production. The fusion protein comprises a SPARC polypeptide and a second polypeptide fused together in frame. The second polypeptide may be a fusion partner known in the art to improve the solubility of the polypeptide to which it is fused, for example, NusA, bacterioferritin (BFR), GrpE, thioredoxin (TRX) and glutathione-S-transferase (GST). Novagen Inc. (Madison, Wis.) provides the pET 43.1 vector series which permit the formation of a NusA-target fusion. DsbA and DsbC have also shown positive effects on expression levels when used as a fusion partner, therefore can be used to fuse with a SPARC polypeptide for achieving higher solubility.

In one embodiment, a SPARC polypeptide is produced as a fusion polypeptide comprising the Q3 SPARC deletion mutant polypeptide and a fusion partner thioredoxin, as described in U.S. Pat. No. 6,387,664, hereby incorporated by reference in its entirety. The thioredoxin-SPARC fusion can be produced in E. coli as an easy-to-formulate, soluble protein in a large quantity without losing the physiological activities. Although U.S. Pat. No. 6,387,664 provides a fusion SPARC protein with SPARC fused to the C-terminus of thioredoxin, it is understood, for the purpose of the present invention, a SPARC polypeptide can be fused either to the N-terminus or the C-terminus of a second polypeptide, so long as its sensitizing function is retained.

The polypeptides of the invention can be also be synthesize in vitro, e.g., using any suitable in vitro translation system, e.g., TNT® Quick Coupled Transcription/Translation Systems (Promega, Madison, WI), rabbit reticulocyte lysates, wheat germ extracts, and the like. Alternatively, the polypeptides made in accordance with the invention may be chemically synthesized by any suitable solid phase or liquid phase protocols including, e.g., Lithographic, Fmoc solid-phase and t-Boc solid-phase peptide synthesis approaches.

By isolated or purified it is meant constituting at least 75% , at least 90%, at least 95% , at least 99% of the polypeptide or polynucleotide present. Polynucleotides in accordance with the invention my be purified by any suitable means. The polypeptides of the invention can be purified by any suitable method know to those of ordinary skill, including, e.g., the methods discussed in Sage: Purification of SPARC/osteonectin, Curr. Protocols Cell Biol. 2003 Feb; Chapter 10: Unit 10.11, which is herby incorporated by reference in its entirety. Alternatively, affinity chromatography or precipitation using any suitable antibody, epitope tags including, e.g., myc, gfp, V5, FITC, HA, S-tag, T7, and the like or other suitable affinity systems may be used, including, e.g., biotin/avidin, polyhistidine/Nickel, GST and the like.

One measure of "correspondence" of nucleic acids, peptides or proteins for use herein with reference to the above described nucleic acids and proteins is relative "identity" between sequences. In the case of peptides or proteins, or in the case of nucleic acids defined according to a encoded peptide or protein correspondence includes a peptide having at least about 50% identity, alternatively at least about 70% identity, alternatively at least about 90% identity, or even about 95% and may also be at least about 98-99% identity to a specified peptide or protein. Preferred measures of identity as between nucleic acids is the same as specified above for peptides with at least about 90% or at least about 98-99% identity being most preferred.

The term "identity" as used herein refers to the measure of the identity of sequence between two peptides or between two nucleic acids molecules. Identity can be determined by comparing a position in each sequence, which may be a line for purposes of comparison. Two amino acid or nucleic acid sequences are considered substantially identical if they share at least about 75% sequence identity, preferably at least about 90% sequence identity and even more preferably at least 95% sequence identity and most preferably at least about 98-99% identity.

Sequence identity may be determined by the BLAST algorithm currently is use and which was originally described in Altschul et al. (1990) J. Mol. Biol. 215:403-410. The BLAST algorithm may be used with the published default settings. When a position in the compared sequence is occupied by the same base or amino acid, the molecules are considered to have shared identity at that position. The degree of identity between sequences is a function of the number of matching positions shared by the sequences.

An alternate measure of identity of nucleic acid sequences is to determine whether two sequences hybridize to each other under low stringency, and preferably high stringency conditions. Such sequences are substantially identical when they will hybridize under high stringency conditions. Hybridization to filter-bound sequences under low stringency conditions may, for example, be performed in 0.5 M NaHPO₄, 7% sodium dodecyl sulfate (SDS), 1 mM EDTA at 65 °C, and washing in 0.2.times.SSC/0. 1 SDS at 42.degree. C. (see Ausubel et al. (eds.) 1989, Current Protocols in Molecular Biology, Vol. 1, Green Publishing Associates, Inc., and John Wiley & sons, Inc., New York, at p. 2.10.3). Alternatively, hybridization to filter-bound sequences under high stringency conditions, may for example, be performed in 0.5 M NaHPO₄, 7% (SDS), 1 mM EDTA at 65 °C, and washing in 0.2 .times.SSC/0.1% SDS at 68.degree. C. (see Ausubel et al. (eds.) 1989, supra). Hybridization conditions may be modified in accordance with known methods depending on the sequence of interest (see Tijssen, 1993, Laboratory Techniques in Biochemistry and Molecular Biology--Hybridization with Nucleic Acid Probes, Part I, Chapter 2 "Overview of Principles in Hybridization and the Strategy of Nucleic Acid Probe Assays", Elsevier, N.Y.). Generally, stringent conditions are selected to be about 5 °C lower than the thermal melting point for the specific sequence at a defined ionic strength and pH.

It will be appreciated by a person of skill in the art that the numerical designations of the positions of mutations within a sequence are relative to the specific sequence. Also the same positions may be assigned different numerical designations depending on the way in which the sequence is numbered and the sequence chosen. Furthermore, sequence variations such as insertions or deletions, may change the relative position and subsequently the numerical designations of particular nucleotides at and around a mutational site.

Gene therapy is a medical intervention that involves modifying the genetic material of living cells to fight disease. Gene therapy is being studied in clinical trials (research studies with humans) for many different types of cancer and for other diseases. Accordingly, the invention further provides for an isolated nucleic acid molecule encoding a SPARC polypeptide suitable for use in "gene therapy" (see, e.g., Patil et al., AAPS J. 7(1):E61-77 (2005)).

In general, a gene is delivered to the cell using a "vector" such as those disclosed herein. The most common types of vectors used in gene therapy are viruses. Viruses used as vectors in gene therapy are genetically disabled; they are unable to reproduce themselves. Most gene therapy clinical trials rely on mouse retroviruses to deliver the desired gene. Other viruses used as vectors include adenoviruses, adeno-associated viruses, poxviruses, and the herpes virus. Suitable viral gene therapy vectors and modes of their administration in vivo and ex vivo are known in the art.

Gene therapy can be performed both ex vivo and in vivo. Typically, in ex vivo gene therapy clinical trials, cells from the patient's blood or bone marrow are removed and grown in the laboratory. The cells are exposed to the virus that is carrying the desired gene. The virus enters the cells, and the desired gene becomes part of the cells' DNA. The cells grow in the laboratory and are then returned to the patient by injection into a vein. Using in vivo gene therapy, vectors such as, e.g., viruses or liposomes may be used to deliver the desired gene to cells inside the patient's body.

Those of ordinary skill in the art will recognize that, because of the universality of the genetic code, the knowledge of any given amino acid sequence allows those of ordinary skill in the art to readily envision a finite number of specific polynucleotide sequences that can encode a polypeptide of said amino acid sequence. Further, the ordinarily skilled artisan can readily determine the optimal polynucleotide sequence to encode a polypeptide of said amino acid sequence for expression in any given species via the process of "codon optimization," which is well know in the art (see, e.g., Villalobos et al.: Gene Designer: a synthetic biology tool for constructing artificial DNA segments. BMC Bioinformatics. 2006 Jun. 6;7:285).

As used herein, a "carrier" refers to any substance suitable as a vehicle for delivering an Active Pharmaceutical Ingredient (API) to a suitable in vitro or in vivo site of action. As such, carriers can act as an excipient for formulation of a therapeutic or experimental reagent containing an API. Preferred carriers are capable of maintaining an API in a form that is capable of interacting with a T cell. Examples of such carriers include, but are not limited to water, phosphate buffered saline, saline, Ringer's solution, dextrose solution, serum-containing solutions, Hank's solution and other aqueous physiologically balanced solutions or cell culture medium. Aqueous carriers MAY also contain suitable auxiliary substances required to approximate the physiological conditions of the recipient, for example, enhancement of chemical stability and isotonicity. Suitable auxiliary substances include, for example, sodium acetate, sodium chloride, sodium lactate, potassium chloride, calcium chloride, sorbitan monolaurate, triethanolamine oleate, and other substances used to produce phosphate buffer, Tris buffer, and bicarbonate buffer.

As used herein "anti-cancer vaccine" means a composition comprising a tumor associated antigen or epitope against which an immune response may be mounted.

In another embodiment, the invention provides an anti-cancer vaccine comprising a peptide antigen having the amino acid sequence shown in SEQ ID NO: 1; or a peptide antigen having an amino acid sequence comprising a substitution, deletion, insertion, and/or addition of one or several amino acids with respect to the amino acid sequence shown in SEQ ID NO: 1, and also having immune-stimulating activity. In another aspect, the present invention provides a peptide antigen comprising a portion of the aforementioned peptide of SEQ ID NO 1 and having immune-stimulating activity. In yet another aspect, the present invention provides a peptide antigen which has an amino acid sequence comprising a substitution, deletion, insertion, and/or addition of one or several amino acids with respect to the aforementioned portion of the peptide antigen of SEQ ID NO 1, and also has immune-stimulating activity. The above-described peptide antigens can preferably activate cytotoxic T lymphocytes which recognize a cancer antigen protein.

In another aspect, the present invention provides helper T cells, cytotoxic T lymphocytes, or an immunocyte population comprising these cells, which are induced by in vitro stimulation using the aforementioned peptide antigens, or a mixture thereof.

In another aspect, the present invention provides helper T cells, cytotoxic T lymphocytes, or an immunocyte population comprising these cells, which are induced by in vitro stimulation using the aforementioned peptide antigens, or a mixture thereof, and an immune activator. The immune activator is preferably a cell growth factor or cytokine.

The vaccine may preferably further comprise an adjuvant, such as complete Freund's adjuvant, incomplete Freund's adjuvant, alum, Bacillus Calmette-Guerin, agonists and modifiers of adhesion molecules, tetanus toxoid, imiquinod, montanide, MPL, and QS21.

In another aspect, the present invention provides a method for suppressing a tumor, which comprises introducing the above-described helper T cells, cytotoxic T lymphocytes, or an immunocyte population comprising these cells into a body. The above-described method is preferably used to prevent and/or treat cancers.

In another aspect, the present invention provides a cell culture solution used to produce the helper T cells or cytotoxic T lymphocytes of the present invention or an immunocyte population comprising these cells, which comprises the aforementioned peptide antigens, or a mixture thereof.

In another aspect, the present invention provides a cell culture kit for producing the helper T cells or cytotoxic T lymphocytes of the present invention or an immunocyte population comprising these cells, which comprises the above-described cell culture solution and a cell culture vessel.

In another aspect, the present invention provides DNA encoding the aforementioned peptide antigens. In yet another aspect, the present invention provides a cancer vaccine, which comprises the aforementioned DNA of the present invention, or recombinant virus or recombinant bacteria comprising the above-described DNA. The above-described cancer vaccine preferably further comprises an adjuvant.

The vaccine may comprise more than one peptide, and the multiple peptides may depend on the tumor to be treated. The vaccine may further comprise an antigen presenting cell, such as a dendritic cell, and more particularly a dendritic cell pulsed or loaded with the peptide and used as a cellular vaccine to stimulate T cell immunity against the peptide, and thereby against the tumor.

The administration of the pharmaceutical compositions of the present invention can be accomplished via any suitable route including, but not limited to, intravenous, subcutaneous, intramuscular, intraperitoneal, intratumoral, oral, rectal, vaginal, intravesical, and inhalational administration, with intravenous and intratumoral administration being most preferred. The composition can further comprise any other suitable components, especially for enhancing the stability of the composition and/or its end use. Accordingly, there is a wide variety of suitable formulations of the composition of the invention. The following formulations and methods are merely exemplary and are in no way limiting.

The pharmaceutical compositions can also include, if desired, additional therapeutic or biologically-active agents. For example, therapeutic factors useful in the treatment of a particular indication can be present. Factors that control inflammation, such as ibuprofen or steroids, can be part of the composition to reduce swelling and inflammation associated with in vivo administration of the pharmaceutical composition and physiological distress.

The carrier typically will be liquid, but also can be solid, or a combination of liquid and solid components. The carrier desirably is physiologically acceptable (e.g., a pharmaceutically or pharmacologically acceptable) carrier (e.g., excipient or diluent). Physiologically acceptable carriers are well known and are readily available. The choice of carrier will be determined, at least in part, by the location of the target tissue and/or cells, and the particular method used to administer the composition.

Typically, such compositions can be prepared as injectables, either as liquid solutions or suspensions; solid forms suitable for using to prepare solutions or suspensions upon the addition of a liquid prior to injection can also be prepared; and the preparations can also be emulsified. The pharmaceutical formulations suitable for injectable use include sterile aqueous solutions or dispersions; formulations containing known protein stabilizers and lyoprotectants, formulations including sesame oil, peanut oil or aqueous propylene glycol, and sterile powders for the extemporaneous preparation of sterile injectable solutions or dispersions. In all cases the formulation must be sterile and must be fluid to the extent that easy syringability exists. It must be stable under the conditions of manufacture and storage and must be preserved against the contaminating action of microorganisms, such as bacteria and fungi. Solutions of the active compounds as free base or pharmacologically acceptable salts can be prepared in water suitably mixed with a surfactant, such as hydroxycellulose. Dispersions can also be prepared in glycerol, liquid polyethylene glycols, and mixtures thereof and in oils. Under ordinary conditions of storage and use, these preparations contain a preservative to prevent the growth of microorganisms.

The peptides of the present invention, can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such as organic acids as acetic, oxalic, tartaric, mandelic, and the like. Salts formed with the free carboxyl groups also can be derived from inorganic bases such as, for example, sodium, potassium, ammonium, calcium, or ferric hydroxides, and such organic bases as isopropylamine, trimethylamine, histidine, procaine and the like.

Formulations suitable for parenteral administration include aqueous and non aqueous, isotonic sterile injection solutions, which can contain anti oxidants, buffers, bacteriostats, and solutes that render the formulation isotonic with the blood of the intended recipient, and aqueous and non aqueous sterile suspensions that can include suspending agents, solubilizers, thickening agents, stabilizers, and preservatives. The formulations can be presented in unit dose or multi dose sealed containers, such as ampules and vials, and can be stored in a freeze dried (lyophilized) condition requiring only the addition of a sterile liquid excipient, for example, water, for injections, immediately prior to use. Extemporaneous injection solutions and suspensions can be prepared from sterile powders, granules, and tablets of the kind previously described. In a preferred embodiment of the invention, the peptide ligand domain-containing conjugate is formulated for injection (e.g., parenteral administration). In this regard, the formulation desirably is suitable for intratumoral administration, but also can be formulated for intravenous injection, intraperitoneal injection, subcutaneous injection, and the like.

The invention also provides, if desirable, embodiments in which the peptides of the present invention are further conjugated to polyethylene glycol (PEG). PEG conjugation can increase the circulating half-life of these polypeptides, reduce the polypeptide's immunogenicity and antigenicity, and improve their bioactivity. If used, any suitable method of PEG conjugation can be used, including but not limited to, reacting methoxy-PEG with a peptide's available amino group(s) or other reactive sites such as, e.g., histidines or cysteinees. In addition, recombinant DNA approaches can be used to add amino acids with PEG-reactive groups to the peptide ligand domain-containing conjugate. Further, releasable and hybrid PEG-ylation strategies can be used in accordance with the aspects of the present invention, such as the PEG-ylation of polypeptide, wherein the PEG molecules added to certain sites in the peptide ligand domain-containing conjugatemolecule are released in vivo. Examples of PEG conjugation methods are known in the art. See, e.g., Greenwald et al., Adv. Drug Delivery Rev. 55:217-250 (2003).

Formulations suitable for administration via inhalation include aerosol formulations. The aerosol formulations can be placed into pressurized acceptable propellants, such as dichlorodifluoromethane, propane, nitrogen, and the like. They also can be formulated as non pressurized preparations, for delivery from a nebulizer or an atomizer.

Formulations suitable for anal administration can be prepared as suppositories by mixing the active ingredient with a variety of bases such as emulsifying bases or water soluble bases. Formulations suitable for vaginal administration can be presented as pessaries, tampons, creams, gels, pastes, foams, or spray formulas containing, in addition to the active ingredient, such carriers as are known in the art to be appropriate.

In addition, the composition of the invention can comprise additional therapeutic or biologically active agents. For example, therapeutic factors useful in the treatment of a particular indication can be present. Factors that control inflammation, such as ibuprofen or steroids, can be part of the composition to reduce swelling and inflammation associated with in vivo administration of the pharmaceutical composition and physiological distress.

In the case of inhalational therapy, the pharmaceutical composition of the present invention is desirably in the form of an aerosol. Aerosol and spray generators for administering the agent if in solid form are available. These generators provide particles that are respirable or inhalable, and generate a volume of aerosol containing a predetermined metered dose of a medicament at a rate suitable for human administration. Examples of such aerosol and spray generators include metered dose inhalers and insufflators known in the art. If in liquid form, the pharmaceutical compositions of the invention can be aerosolized by any suitable device.

When used in connection with intravenous, intraperitoneal or intratumoral administration, the pharmaceutical composition of the invention can comprise sterile aqueous and non-aqueous injection solutions, suspensions or emulsions of the active compound, which preparations are preferably isotonic with the blood of the intended recipient. These preparations can contain one or more of anti-oxidants, buffers, surfactants, cosolvents, bacteriostats, solutes which render the compositions isotonic with the blood of the intended recipient, and other formulation components known in the art. Aqueous and non-aqueous sterile suspensions can include suspending agents and thickening agents. The compositions can be presented in unit-dose or multi-dose containers, for example sealed ampoules and vials.

The methods of the present invention can also be part of combination therapy. The phrase "combination therapy" refers to administering a therapeutic agent in accordance with the invention together with another therapeutic composition in a sequential or concurrent manner such that the beneficial effects of this combination are realized in the mammal undergoing therapy. Optimal dosages for any of the compositions of the invention can be determined by routine methods known to those ordinary skill in the art.

Methods for coupling or conjugation of suitable diagnostics therapeutics, chemotherapeutics, radionuclides, polypeptides, and the like to antibodies or fragments thereof are well described in the art. For example, The invention provides for a SPARC-binding polypeptide, SPARC polypeptide or anti-SPARC polypeptide antibodies conjugates, such as, e.g., SPARC-radioinuclide, SPARC-drug, SPARC-immunomodulator or SPARC-toxin conjugates. Any suitable method can be used in accordance with the invention to form the polypeptide conjugates. For example, without limitation, free amino groups in SPARC proteins, such the epsilon-amino group of lysine, can be conjugated with reagents such as carodiimides or heterobiofunctional agents. Alternatively, e.g., polypeptide suflhydryl groups can be used for conjugation. In addition, sugar moieties bound to glycoproteins or an anti-SPARC antibodies, e.g., anti-SPARC polypeptide antibodies can be oxidized to form aldehydes groups useful in a number of coupling procedures known in the art. The conjugates formed in accordance with the invention can be stable in vivo or labile, such as enzymatically degradeable tetrapeptide linakages or acid-labile cis-aconityl or hydrazone linkages. In addition, nucleic acids encoding suitable fusion proteins could be employed to generate coupled polypeptide, e.g., GFP or toxin fusion proteins.

### EXAMPLE 1

This example demonstrates the interaction of SPARC and Abraxane® with the antiangiogenic agent Sutent® In a PC3 model. Tumor volume was measured in mice being treated with Abraxane alone (15 mg/kg administered daily for five days), Abraxane and Sutent (the Sutent administered at 30 mg/kg daily for 8 weeks), Abraxane and exogenous SPARC (the SPARC at 0.2 mg/ms administered twice a week for eight weeks), and finally, Abraxane, Sutent and SPARC together.

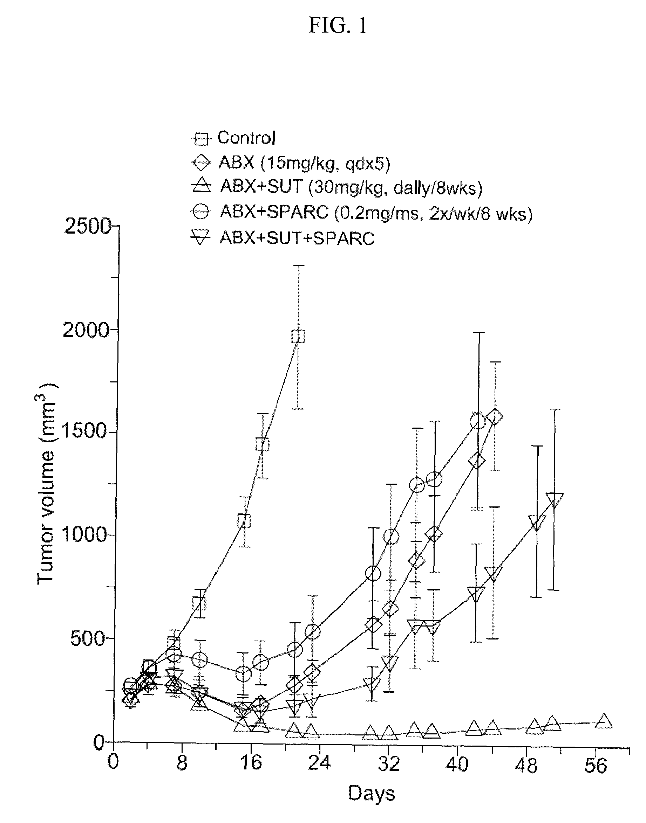

FIG. 1 depicts a graph plotting tumor volume (mm³) against time (days) for these test conditions. As is apparent from the graph, the administration of Abraxane® results in markedly lower tumor volume, relative to control, over the course of the experiment. When Abraxane® is administered with SPARC tumor volume is slightly greater indicating (as was shown in example 1) that exogenously administered SPARC desensitizes Abraxane® in this system. When the antiangiogenic agent Sutent® is administered along with SPARC and Abraxane®, the efficacy of the SPARC/Abraxane® combination is markedly improved.

FIG. 1 also demonstrates that the administration of Abraxane® with the anti-angiogenic agent Sutent® produces a far greater decrease in tumor volume than the administration of Abraxane® alone. Surprisingly, the administration of exogenous SPARC along with Abraxane® and Sutent® negates some of the synergistic affect of Abraxane® and Sutent®. This suggests that SPARC antagonizes the anti-angiogenic activity of Sutent®.

These data suggest that the mechanism by which SPARC desensitizes these particular anti-tumor agents is via angiogenic activity.

### EXAMPLE 2

This example demonstrates the characterization of the angiogenic behavior of SPARC.

Recombinant human SPARC and genetically engineered variants were expressed and purified using HEK 293 cells maintained in hollow fiber bio-reactors. The angiogenic activity of rhSPARC and its variants was evaluated using a HUVEC tube formation assay and a HUVEC sprout formation bead assay.

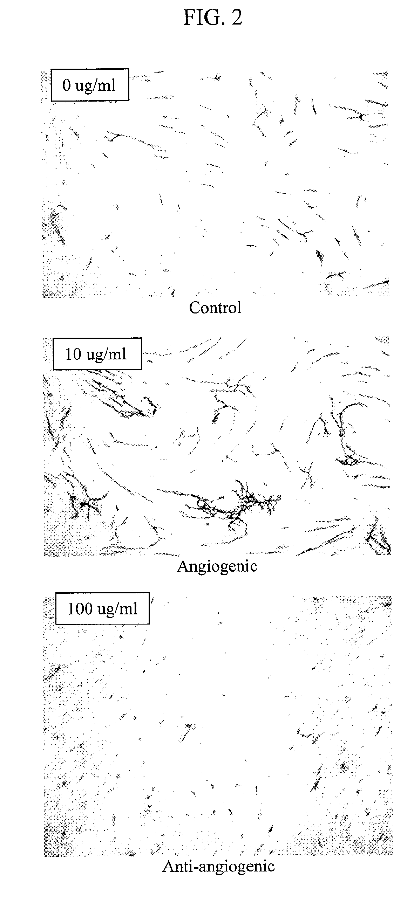

In the HUVEC tube formation assay, rhSPARC was pro-angiogenic at 10 µg/mL and anti-angiogenic at 100 µg/mL. The results of the tube formation assay can be seen in FIG. 2 In the sprout formation assay, addition of rhSPARC resulted in more mature blood vessels well supported by pericytes, suggesting a role for SPARC beyond the initial stimulation of angiogeneis per se. Additional rhSPARC variants with deletions and single/double amino acid substitutions tested in these assays included: Q3 deletion (BIO2), an inversion of the putative angiogenic domain (BIO5), a double K>Q substitution in the proposed angiogenic domain (BIO11), the genetic ablation of the putative catephsin K recognition sites (BIO8) and a proteolytic degradation product of rhSPARC. Terminal amino acid analysis indicated the angiogenic activity is localized to SEQ ID NO: 1.

### EXAMPLE 3

This example depicts the identification of the angiogenic domain of SPARC.

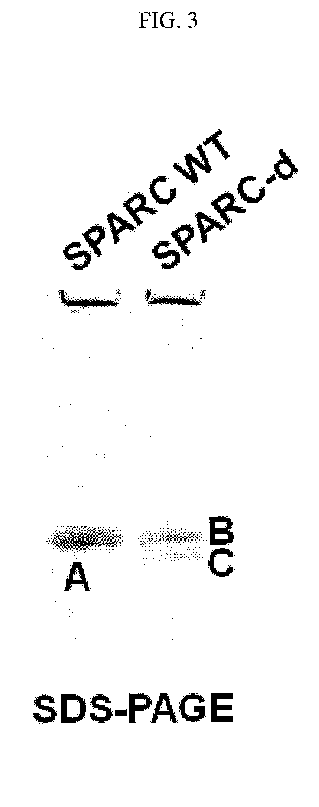

A proteolytic degradation product of SPARC was prepared and designated SPARC-d. SPARC-d is a mixture consisting of two forms of C-terminal truncated SPARC. FIG. 3 depicts an SDS PAGE assay in which SPARC d was run alongside wildtype SPARC. The dominant form of SPARC-d, labeled B on the gel in FIG. 3, is missing part of the C-terminal sequence consisting of amino acids 233-286 (SEQ ID NO. 2)

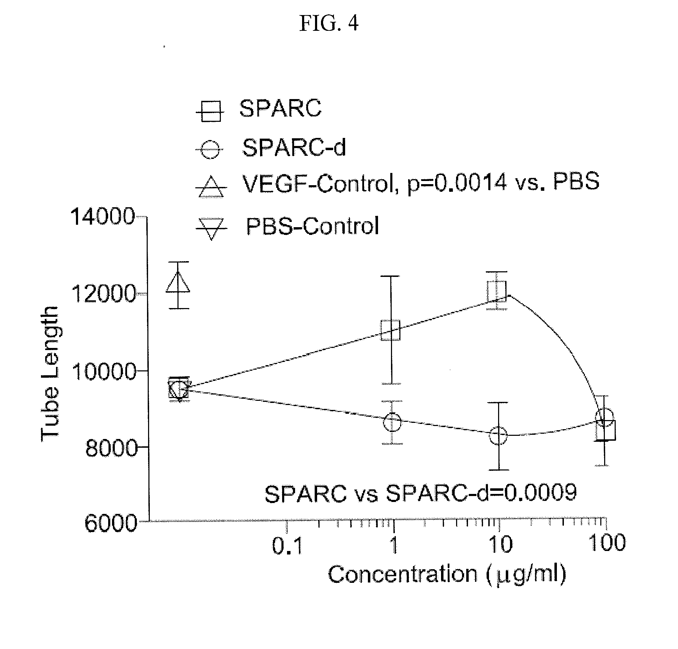

FIG. 4 depicts the results of a HUVEC 3-D tube formation assay conducted with wild type SPARC and SPARC-D. The angiogenic behavior of wild type SPARC, as described in the previous example, can be seen in the graph; the angiogenic behavior increases as the concentration approaches 10 ug/ml and drops off as it approaches 100 ug/ml. The results for SPARC-d, however, indicate that truncating the C-terminal end of the protein abolishes SPARC angiogenic activity.

Based on the results of this assay, it is possible to identify the location of the angiogenic domain of SPARC as located within the C-Terminal 54 amino acid sequence (SEQ ID NO 1).

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

Aspects of the invention are:
1. A method of treating an animal suffering from a SPARC-dependent disease with a therapy comprising:
   (a) quantifying the amount of SPARC angiogenic domain polypeptides and full length SPARC comprising the SPARC angiogenic domain at a disease site in said animal,
   (b) quantifying the amount of SPARC angiogenic domain polypeptides and full length SPARC comprising the SPARC angiogenic domain at a disease site in one or more other animals suffering from the same SPARC dependent disease which are known to respond to the therapy,
   (c) calculating the average of the amounts of SPARC angiogenic domain polypeptides and full length SPARC comprising the SPARC angiogenic domain determined in (b);
   (d) comparing said amount determined in (a) to said average determined in (c), and
   (e) administering the therapy if the amount determined in (a) is greater than or equal to the average determined in (c).
2. A method of treating an animal suffering from a SPARC-dependent disease with a therapy comprising:
   (a) quantifying the amount of SPARC angiogenic domain polypeptides at a disease site in said animal,
   (b) quantifying the amount of SPARC angiogenic domain polypeptides at a disease site in one or more other animals suffering from the same SPARC dependent disease as the animal in (a) which are known to respond to the therapy,
   (c) calculate the average of the amounts of SPARC angiogenic domain polypeptides determined in (b);
   (d) comparing said amount determined in (a) to the average amount of SPARC angiogenic domain polypeptides determined in (c), and
   (c) administering the therapy if the amount determined in (a) is greater than or equal to the average of the amounts determined in (b).
3. The method of either of items 1 or 2, wherein the SP ARC-dependent disease is a tumor, hyperthrophic scar, keloid, or endometriosis, or diabetic retinopathy.
4. The method of either of items 1 or 2, wherein the therapy is one or more of a chemotherapeutic, radiation or biologic regimen.
5. A method of treating an animal with a SPARC dependent condition or disease comprising administering to the animal a therapeutically effective amount a polypeptide which binds to a polypeptide of comprising the SPARC angiogenic domain and concentrates at a disease site.
6. The method of item 5, wherein polypeptide is conjugated to a chemotherapeutic, radiation or biologic agent.
7. The method of either item 5 or 6, wherein the polypeptide is an antibody.
8. A method of treating an animal with a SPARC dependent condition or disease comprising inoculating the animal with an immunologically effective amount of an immunogen comprising a SPARC angiogenic domain.
9. The method of any one of items 1-8, wherein the animal is a human.

## Claims

1. A polypeptide which binds to a polypeptide comprising the SPARC angiogenic domain and concentrates at a disease site for use in treating an animal with a SPARC dependent condition or disease.

2. The polypeptide for use of claim 1, wherein the polypeptide is conjugated to a chemotherapeutic, radiation or biologic agent.

3. The polypeptide for use of claim 1 or 2, wherein the polypeptide is an antibody.

4. An immunogen comprising a SPARC angiogenic domain for use in treating an animal with a SPARC dependent condition or disease.

5. The polypeptide for use of any one of claims 1 to 3, wherein the animal is a human.

6. The immunogen for use of claim 4, wherein the animal is a human.


SEQUENCE LISTING

<110>  ABRAXIS BIOSCIENCE, LLC

<120>  SPARC ANGIOGENIC DOMAIN AND METHODS OF USE

<130>  EP84039IFZ210pau

<140>  not yet assigned
<141>  2011-03-11

<150>  EP11754152.4
<151>  2011-03-11

<150>  PCT/US2011/028080
<151>  2011-03-11

<150>  61/313,050
<151>  2010-03-11

<150>  61/313,047
<151>  2010-03-11

<160>  3

<170>  PatentIn version 3.5

<210>  1
<211>  54
<212>  PRT
<213>  Homo sapiens

<400>  1

Thr Glu Leu Ala Pro Leu Arg Ala Pro Leu Ile Pro Met Glu His Cys
1               5                   10                  15

Thr Thr Arg Phe Phe Glu Thr Cys Asp Leu Asp Asn Asp Lys Tyr Ile
            20                  25                  30

Ala Leu Asp Glu Trp Ala Gly Cys Phe Gly Ile Lys Gln Lys Asp Ile
        35                  40                  45

Asp Lys Asp Leu Val Ile
    50

<210>  2
<211>  232
<212>  PRT
<213>  Homo sapiens

<400>  2

Ala Pro Gln Gln Glu Ala Leu Pro Asp Glu Thr Glu Val Val Glu Glu
1               5                   10                  15

Thr Val Ala Glu Val Thr Glu Val Ser Val Gly Ala Asn Pro Val Gln
            20                  25                  30

Val Glu Val Gly Glu Phe Asp Asp Gly Ala Glu Glu Thr Glu Glu Glu
        35                  40                  45

Val Val Ala Glu Asn Pro Cys Gln Asn His His Cys Lys His Gly Lys
    50                  55                  60

Val Cys Glu Leu Asp Glu Asn Asn Thr Pro Met Cys Val Cys Gln Asp
65                  70                  75                  80

Pro Thr Ser Cys Pro Ala Pro Ile Gly Glu Phe Glu Lys Val Cys Ser
                85                  90                  95

Asn Asp Asn Lys Thr Phe Asp Ser Ser Cys His Phe Phe Ala Thr Lys
            100                 105                 110

Cys Thr Leu Glu Gly Thr Lys Lys Gly His Lys Leu His Leu Asp Tyr
        115                 120                 125

Ile Gly Pro Cys Lys Tyr Ile Pro Pro Cys Leu Asp Ser Glu Leu Thr
    130                 135                 140

Glu Phe Pro Leu Arg Met Arg Asp Trp Leu Lys Asn Val Leu Val Thr
145                 150                 155                 160

Leu Tyr Glu Arg Asp Glu Asp Asn Asn Leu Leu Thr Glu Lys Gln Lys
                165                 170                 175

Leu Arg Val Lys Lys Ile His Glu Asn Glu Lys Arg Leu Glu Ala Gly
            180                 185                 190

Asp His Pro Val Glu Leu Leu Ala Arg Asp Phe Glu Lys Asn Tyr Asn
        195                 200                 205

Met Tyr Ile Phe Pro Val His Trp Gln Phe Gly Gln Leu Asp Gln His
    210                 215                 220

Pro Ile Asp Gly Tyr Leu Ser His
225                 230

<210>  3
<211>  303
<212>  PRT
<213>  Homo sapiens

<400>  3

Met Arg Ala Trp Ile Phe Phe Leu Leu Cys Leu Ala Gly Arg Ala Leu
1               5                   10                  15

Ala Ala Pro Gln Gln Glu Ala Leu Pro Asp Glu Thr Glu Val Val Glu
            20                  25                  30

Glu Thr Val Ala Glu Val Thr Glu Val Ser Val Gly Ala Asn Pro Val
        35                  40                  45

Gln Val Glu Val Gly Glu Phe Asp Asp Gly Ala Glu Glu Thr Glu Glu
    50                  55                  60

Glu Val Val Ala Glu Asn Pro Cys Gln Asn His His Cys Lys His Gly
65                  70                  75                  80

Lys Val Cys Glu Leu Asp Glu Asn Asn Thr Pro Met Cys Val Cys Gln
                85                  90                  95

Asp Pro Thr Ser Cys Pro Ala Pro Ile Gly Glu Phe Glu Lys Val Cys
            100                 105                 110

Ser Asn Asp Asn Lys Thr Phe Asp Ser Ser Cys His Phe Phe Ala Thr
        115                 120                 125

Lys Cys Thr Leu Glu Gly Thr Lys Lys Gly His Lys Leu His Leu Asp
    130                 135                 140

Tyr Ile Gly Pro Cys Lys Tyr Ile Pro Pro Cys Leu Asp Ser Glu Leu
145                 150                 155                 160

Thr Glu Phe Pro Leu Arg Met Arg Asp Trp Leu Lys Asn Val Leu Val
                165                 170                 175

Thr Leu Tyr Glu Arg Asp Glu Asp Asn Asn Leu Leu Thr Glu Lys Gln
            180                 185                 190

Lys Leu Arg Val Lys Lys Ile His Glu Asn Glu Lys Arg Leu Glu Ala
        195                 200                 205

Gly Asp His Pro Val Glu Leu Leu Ala Arg Asp Phe Glu Lys Asn Tyr
    210                 215                 220

Asn Met Tyr Ile Phe Pro Val His Trp Gln Phe Gly Gln Leu Asp Gln
225                 230                 235                 240

His Pro Ile Asp Gly Tyr Leu Ser His Thr Glu Leu Ala Pro Leu Arg
                245                 250                 255

Ala Pro Leu Ile Pro Met Glu His Cys Thr Thr Arg Phe Phe Glu Thr

260                 265                 270

Cys Asp Leu Asp Asn Asp Lys Tyr Ile Ala Leu Asp Glu Trp Ala Gly
        275                 280                 285

Cys Phe Gly Ile Lys Gln Lys Asp Ile Asp Lys Asp Leu Val Ile
    290                 295                 300